(19) Europäisches Patentamt
European Patent Office
Office européen des brevets

(11) **EP 4 736 850 A1**

(12) **EUROPEAN PATENT APPLICATION**

(43) Date of publication:
**06.05.2026 Bulletin 2026/19**

(21) Application number: **24383193.0**

(22) Date of filing: **31.10.2024**

(51) International Patent Classification (IPC):
***A61K 31/194*** (2006.01) ***A23L 33/00*** (2016.01)
***A61K 9/00*** (2006.01) ***A61K 31/198*** (2006.01)
***A61K 31/375*** (2006.01) ***A61K 31/401*** (2006.01)
***A61P 3/02*** (2006.01) ***A61P 21/00*** (2006.01)
***A61P 43/00*** (2006.01)

(52) Cooperative Patent Classification (CPC):
(C-Sets available)
**A61K 31/198; A23L 33/175; A61K 31/194; A61K 31/375; A61K 31/401; A61P 3/02; A61P 21/00; A61P 43/00;** A61K 9/0014 (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
- **Melendez Hevia, Enrique**
  **38006 Tenerife (ES)**
- **Matos Expósito, Gustavo Adolfo**
  **38006 Tenerife (ES)**

(72) Inventors:
- **Melendez Hevia, Enrique**
  **38006 Tenerife (ES)**
- **Matos Expósito, Gustavo Adolfo**
  **38006 Tenerife (ES)**

(74) Representative: **Clarke, Modet y Cía., S.L.**
**C/ Suero de Quiñones 34-36**
**28002 Madrid (ES)**

(54) **USE OF A COMBINATION OF GLYCINE, L-PROLINE, L-LYSINE, L-ASPARTIC ACID, CITRIC ACID AND VITAMIN C, IN THE PREVENTION OF HEALTH AND PERFORMANCE CONDITIONS IN PHYSICAL EXERCISES IN A SUBJECT**

(57) The present invention is a method to improve physical-sports performance, reduce fatigue, combat physical-sports aging, and to cure and prevent physical injuries, based on an effective dose of glycine, *L*-proline, *L*-lysine, *L*-aspartic acid (or *L*-aspartate, citric acid, or citrate), and vitamin C. The method is based on the limitations and subsequent conditions of the metabolism in three aspects: (1) the synthesis of ATP in mitochondria, dependent on the Krebs cycle; (2) the synthesis of proteins of the passive mechanical system (collagen and elastin); (3) the mechanism of muscle contraction in actin-myosin interaction cross-bridge cycles. while ensuring that the diet must contain a sufficient amount of protein.

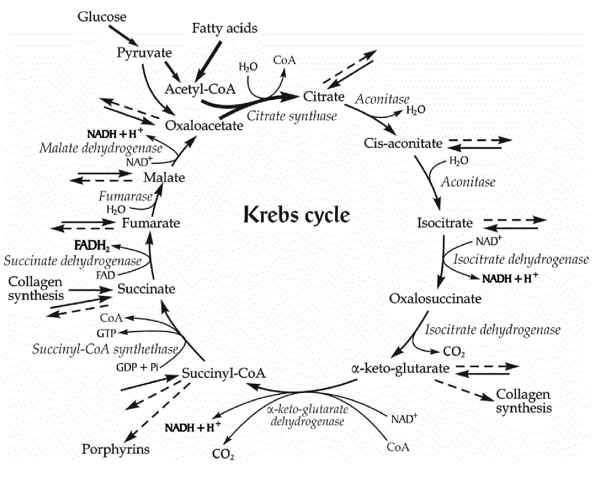


Figure 1



EP 4 736 850 A1

(52) Cooperative Patent Classification (CPC): (Cont.)

C-Sets
**A61K 31/194, A61K 2300/00;**
**A61K 31/198, A61K 2300/00;**
**A61K 31/375, A61K 2300/00;**
**A61K 31/401, A61K 2300/00**

## Description

## FIELD OF INVENTION

The present invention is of the health filed and relates to novel therapeutic and non-therapeutic uses for glycine, *L*-proline, *L*-lysine, vitamin C, *L*-aspartic acid (or citric acid, or their $K^+$-$Mg^{2+}$salts), and vitamin C.

## BACKGROUND

We must begin by pointing out that the objective of what we propose here is not simply an increase in physical fitness, but rather to achieve a comprehensive result on the mechanical and energetic system of the muscle, which includes the improvement of physical fitness, the repair and prevention of injuries, and the combat or delay of sports aging.

In turn, we consider physical fitness - and its improvement - as a complex function that includes five variables: strength (s), endurance (e), velocity (v), achievements-effectiveness (a), and minimizing the discomfort (d), which is a sign of injury.

Therefore, the background where any of these aspects have been studied separately, omitting others, is not significant for our proposal, since it is useless, for example, to increase resistance if this leads to a loss of strength, speed, or aim, just as it is useless to increase resistance if it leads to long-lasting fatigue that leaves the athlete useless for several days or weeks.

### Energy machinery - The Krebs cycle

The Krebs cycle, shown in Figure 1, is the central pathway of metabolism, as all its intermediaries and substrates interact with other routes, and all the routes are more or less closely related to their substrates or intermediaries. Its main quantitative function is the aerobic production of ATP, being the main source of the reduction equivalents (NADH and FADH2) that enter the respiratory chain to produce ATP.

### Anaplerosis - Cataplerosis

The external supply of oxaloacetate or any intermediate of the cycle is called anaplerosis (filling priming or feeding) and the opposite cataplerosis (drainage); these two types of processes participate together in the regulation of the activity of the Krebs cycle. Figure 1 shows the intermediates that are sites of anaplerosis and cataplerosis. Anaplerosis of the Krebs cycle is, therefore, an ideal way of increasing its activity, rather than increasing the concentration of the substrate AcCoA, or any of its sources.

Anaplerosis and cataplerosis are opposite processes that can work in coordination to regulate the activity of the Krebs cycle. However, there are cataplerotic processes whose function is not this regulation but are imposed by the need to synthesize specific products. For the cases at hand, the synthesis of porphyrins from succinyl-CoA, and the hydroxylations of proline and lysine residues in the synthesis of collagen from a-ketoglutarate are highlighted.

### Injuries

There is a mutual interaction between these two concepts: lack of fitness leads to injuries, and injuries are an important cause of decreased fitness; here we are not referring to traumatic injuries, whose loss of fitness is obvious, but to injuries not noticed as such, which we will discuss below.

It should be noted that, apart from the health and well-being of the individual, all of this has a great economic interest, especially in professional athletes, and in jobs that are highly dependent on physical fitness. such as acrobats, film extras, police officers, firemen, etc.

Medical treatments for traumatic injuries have improved greatly in recent years, especially in surgery and physiotherapy, but they seem to have reached a point where no further progress is expected, with recovery times in many cases taking months or years. Apart from personal injury, injuries in this area mean a huge financial loss for the club (and in many cases for the athlete), which can be the biggest expense in the budget.

On the other hand, there is little to say about prevention, where very little progress has been made, apart from avoiding falls and collisions, which is impossible in high-level contact sports. This invention is not just a matter of curing injuries, but of avoiding them. Good fitness should mean not only performing well but avoiding injuries. We distinguish three types of injuries: traumatic injuries, and injuries caused by over-exertion that leads to tiredness and fatigue.

### 2 (a) Traumatic injuries

When talking about physical injuries, we always think of traumatic injuries because the injury is identified with an accident, looking for an external cause.

Traumatic injuries are frequent. They may be accidental, but in sportsmen - especially in contact sports, such as football, American football, rugby, and combat sports - injuries are extremely frequent because players are continually exposed to them; in amateur sportsmen (skiing, paddle tennis, and Canarian wrestling, among others) many injuries occur because they are not well prepared physically, or who are forced to overexert themselves due to a lack of good technical preparation. They are also frequent in sports that do not involve contact, and in non-sportsmen, generally, due to falls, or bad movements.

A main cause of injuries is a weakness of the passive mechanical system (collagen and elastin).

### Collagen

Collagen, the main responsible for the strength of the passive mechanical system, is the most abundant protein in the body (»30% of total proteins). A 70 kg man has 3.72 kg of collagen. Its structure is very special for the abundance of two amino acids: glycine (33.33% of the residues, and 25% of its mass), and proline (25% of the residues and 28.78% of its mass).

A second special feature is that 36.4% of the proline residues are hydroxylated. Lysine is also noteworthy, although it is not very abundant (2.70% of the residues, and 3.95% of its mass), 50% of them are also hydroxylated. All these hydroxylations (necessary to achieve the folding of the triple helix) occur after the residue has been incorporated into the chain, since there are no codons for the hydroxy-aminoacyl-derivatives.

Figure 2 shows the collagen synthesis process. The synthesized monomer (tropocollagen) with the correct proline and lysine hydroxylations forms the triple helix (procollagen), which is exported to the extracellular matrix; There, the telopeptides (heads and tails) are removed, forming collagen microfibrils, which join together to form the definitive collagen.

Collagen fibers are formed by covalent bonds of microfibrils, each of which is the triple helix of a trimer of »1,000 residues (MW »100,000) per monomer, which associate to form the triple helix. However, a certain proportion of tropocollagen peptides are unable to form the triple helix because they do not have the correct configuration. These peptides are then degraded inside the cell to attempt their synthesis again. This process of synthesis-degradation of the newly synthesized tropocollagen constitutes the tropocollagen cycle (or procollagen cycle) [4, 5], which acts as a quality control by checking the correct hydroxylations. Surprisingly, the total yield of correct synthesis is very low, with a very high waste, which affects the majority (from 75% in cartilage, to 90% in bone and heart).

Considering the collagen content of each tissue, its synthesis period to renew it, and the percentage of the cycle, the result is an average of »89% of poorly synthesized collagen. Thus, as a consequence of this waste, a 70 kg man synthesizes 920 g of collagen every day between all tissues, to obtain 96.5 useful grams [4].

This deficient collagen synthesis causes weakness of the passive mechanical system, causing poor physical fitness, a propensity to suffer injuries, and difficulty in repairing them.

Due to the tropocollagen cycle, the synthesis consumes a large amount of glycine that is not satisfied by the diet, nor by its metabolic synthesis [4]. But large amounts of proline and lysine are also used, since their hydroxylated residues are not recycled, but are degraded into other products. It is therefore important to know the cause of the cycle, and how it could be reduced. This high error is not considered due to errors in the synthesis of the peptide, since the error in translation is very low, such as $10^{-4}$ - $10^{-5}$ per residue in the aa-tRNA synthetases [6], but to defects in the hydroxylations. Thus, the cause must be a lack of synchronization between the synthesis of the peptide and the hydroxylations, because one of the two processes (not necessarily the same in each step) is slower than the other, thus producing improper hydroxylations or lack of them.

### Glycine requirement

Since its discovery and its metabolism in the 1820s, glycine has been considered non-essential, as human and animal metabolism can synthesize it. However, it has been demonstrated [7] that its synthesis capacity has an insurmountable limit, because of strong stoichiometric reasons. This limit is insufficient to meet metabolic demands (especially for collagen synthesis), resulting in a deficiency of ten grams per day in a 70 kg man [4]. This makes glycine 'essential' (indispensable). In previously published works [5,8] we have demonstrated this need in chondrocytes cultured in vitro, and thus we have recommended the intake of glycine as a food supplement in doses of 10 g/day, divided into two doses spaced 12 hours apart to fight osteoarthritis, without any mention to physical fitness or repair injuries.

### Proline requirement

Like glycine, proline is considered a non-essential amino acid, as our metabolism can synthesize it from glutamate or ornithine. However, several authors have warned that its supplementation in the diet is necessary for growth. Regarding that explained above, the cause of this need should be the procollagen cycle, whose low efficiency consumes a large amount of it.

Hydroxylations are catalyzed by proline hydroxylase, which uses oxygen and a-keto-glutarate, and vitamin C as substrates.

These hydroxylations are very complex, since only a certain percentage of the prolines (9.1% of collagen residues) must be hydroxylated, leaving the rest (13%) as simple proline, and they must be carried out in specific proline residues, not in any of them.

Although proline is considered a non-essential amino acid, as our metabolism can synthesize it, its capacity for biosynthesis must have a limit, making it conditionally essential, as has been pointed out [9].

### Lysine requirement

Although lysine is not a particularly abundant amino acid in collagen (2.7% of residues), 50% of them are also hydroxylated, which is essential for the formation of the triple helix. In the tropocollagen cycle, all hydroxylated lysine residues are therefore lost, which increases lysine need.

### Hydroxylations in collagen synthesis and the Krebs cycle

Hydroxylations of proline and lysine occur in cytoplasmic vesicles. The synthesis of a-keto-glutarate (aKG), co-substrate of hydroxylases, occurs mainly from the Krebs cycle. As hydroxylations yield succinate as a product, which is reincorporated into the Krebs cycle (Fig. 2), they are not a strictly cataplerotic process, but a bypass of the cycle, omitting the succinyl-CoA synthetase step; this means damage to porphyrin synthesis and a 25% decrease in reduction equivalents (NADH) with the respective consequences in ATP synthesis. Therefore, improving collagen synthesis by reducing the extent of the tropocollagen cycle will not only increase the strength of the mechanical system, but will also do so for the energy function of the Krebs cycle. This must be corrected to ensure the supply of ATP, especially for physical exercise.

### Elastin

Elastin is the protein mainly responsible for the elasticity of tissues (muscle, heart, skin, arteries, etc.). Its synthesis is equally complex, like that of collagen, with the same waste, the consequent recycling and the expenditure of substrates. Although its synthesis is much lower than that of collagen, its need for substrate use is the same, since it depends on aa-tRNAs (gly, pro, lys, etc.). Therefore, the deficiency of glycine, proline and lysine seen above for collagen synthesis also affects the synthesis of elastin, whose deficiency will decrease the elasticity of the tissues, an indispensable condition for physical exercise and for many other properties of the body, such as the skin on the joints, or the mechanical activity of the arteries and the heart.

### (1b) Injuries of the contractile system

However, there are other types of physical injuries, associated with physical fitness and loss of effectiveness, which, although they have not been traditionally considered as such, modern studies are showing that they really are. We are referring to tiredness during exercise (tiredness), and fatigue in the following days (fatigue), and, in general, to the lack of physical fitness, in all its aspects, that accompanies these problems.

Excessive physical exercise in long-duration competitions, such as marathons (more than 4 hours), tennis and paddle tennis (which can be up to 3 hours, and sometimes more), football (90 minutes or 120 minutes with extra time), produces two different effects: (a) tiredness during the match, and (b) fatigue for several days afterwards.

### Tiredness

Tiredness is a feeling of lack of strength during exercise, which decreases physical capacity in all its aspects. Tiredness is recognized as a pathology, although 'mild' and transitory [13, 14]. However, it can be serious, or a warning of a serious problem.

In these conditions, the athlete should notice his diminished strength, and even pain [13-14], which is a warning that there is a problem, and he should stop the effort. But if he persists in this activity, the situation will deteriorate more and

more, and can lead to a collapse, including the cardiac fibers, which explains the heart attack with death, which, unfortunately, occurs too frequently in long and medium duration sports (football, racing, and even basketball). That this situation can lead to death shows that fatigue is a pathology, or a warning of a pathology, which can be greatly aggravated by persisting in exercise in such conditions.

Tiredness is attributed to a lack of ATP for the activity of myosin ATPase, the motor of the contractile mechanism. However, this consideration is not correct, as this enzyme has a very high affinity for ATP ($K_M$ myosin-ATPase = 8 mM). [15].

The normal concentration of ATP in the muscle is 1.0-2.0 mM. During exhaustive exercise, ATP can be consumed at a faster rate than it is replenished, and its concentration can drop significantly; but even so, even if the ATP concentration drops significantly, contraction (ATPase activity) can occur without major differences, since the low $K_M$ of myosin for ATP makes it reach a saturation of »98% with 0.5 mM of ATP, which is insignificant for a significant activity of the ATPase.

Assuming that the cause is a decrease in the concentration of ATP, this alone cannot explain a decrease in the activity of the ATPase. The real cause is more complex. The key is that muscle requires ATP not only for contraction, but also for relaxation and maintenance of the resting state [16].

In reality, tiredness - and all the effects derived from it - is caused by a shortage of ATP, but not for the activity of the ATPase, but to restore the structure of the sarcomere in the contraction cycles, which is much slower than the reaction [17] and requires a higher concentration of ATP than for the activity of the ATPase [16,18].

The shortage of ATP therefore produces damage to the structure of the sarcomere, and it is obvious that prolonged exercise until exhaustion, with a lot of ATP expenditure without replenishment, causes an increase in the damage to the contractile apparatus, making this pathology more serious.

**Mechanism of sarcomere contraction**

The sarcomere (Fig. 3) is the basic contractile unit of the muscle, delimited by two Z discs, and functions as two symmetrical halves starting from the central M band. In each half, the sarcomere has 90 thin actin filaments (arranged hexagonally around the myosin filaments), 30 thick myosin filaments, and the same number of titin molecules (also called connectin), which joins the myosin filaments with the Z discs; titin is an elastic protein that acts as a spring to balance the arrangement of the components of the sarcomere.

TN, TM, $Ca^{2+}$

The current model is shown in Figure 4, which represents a very simplified half sarcomere, with only one myosin head. The contraction is triggered by the release of calcium from the sarcoplasmic reticulum, as a consequence of the nervous stimulus. $Ca^{2+}$ ions interact with troponin (TN), causing tropomyosin (TM) to stop blocking the actin of the thin filament (1), allowing its interaction with the myosin heads of the thick filament (2). The myosin ATPase, activated by its interaction with actin, binds to ATP (3). The reaction ATP + $H_2O$ ® ADP + Pi occurs, with a rotation of the myosin head of »60°. The rotation of a myosin head causes the actin filament to advance »18 nm, producing the sliding of the actin filament (4), and a shortening of the sarcomere of » »80 and 150 times.

However, once the myosin head has rotated, and the actin filament has been displaced (State e), the myosin head does not dissociate from actin, but remains attached to it in a state called Rigor cross-bridge, indicated in grey in the figure (State f), and its association with ATP is required for relaxation (transition from State ***f*** to State ***g***). The formation, persistence and functionality of these rigor states (between 100 and 300 between the two halves of the thick filament) have not been well elucidated. In the next section (Invention) we propose a model that explains this.

The simple mechanism in Figure 4 poses a problem: When the myosin head relaxes to attack the actin filament at a point closer to the Z-disk (States ***g***, ***h***), the actin filament, fixed to the Z-disk, would become free and return to the initial state, induced by the Titin spring (State ***i***), so that next advance is not possible without holding the previous one. In the next section (Invention) we present a model that solves this problem and explains the mechanism of contractile apparatus injuries.

**Fatigue**

Unlike tiredness, fatigue is a lack of strength after exercise, which prevents normal activity. It is a more persistent state, which takes several days (or even weeks) to recover.

The pathology of fatigue is clear, as there is well-recognized muscle damage. This has been extensively studied, pointing out the various pathologies associated with fatigue, including muscle damage, and the molecular mechanism. See, e.g., Refs. [19-24], among others.

**Hydroxylations in collagen synthesis and the Krebs cycle**

Hydroxylations of proline and lysine occur in cytoplasmic vesicles (Fig. 5). The synthesis of a-keto-glutarate

(aKG), co-substrate of hydroxylases, occurs mainly from the Krebs cycle. As hydroxylations yield succinate as a product, which is reincorporated into the Krebs cycle, they are not a strictly cataplerotic process, but a bypass of the cycle, omitting the succinyl-CoA synthetase step; this means damage to porphyrin synthesis (and a 25% decrease in reduction equivalents (NADH) with the respective consequences in ATP synthesis.

**Elastin**

Elastin is the protein mainly responsible for the elasticity of tissues (muscle, heart, skin, arteries, etc.). Its synthesis is equally complex, like that of collagen, with the same waste, the consequent recycling and the expenditure of substrates. Although its synthesis is much lower than that of collagen, its need for substrate use is the same, since it depends on aa-tRNAs (gly, pro, lys, etc.). Therefore, the deficiency of glycine, proline and lysine seen above for collagen synthesis also affects the synthesis of elastin, whose deficiency will decrease the elasticity of the tissues, an indispensable condition for physical exercise and for many other properties of the body, such as the skin on the joints, or the mechanical activity of the arteries and the heart.

**Maintenance of the energy system - Synthesis of porphyrins**

The supply of ATP by the mitochondria does not depend only on the proper functioning of the Krebs cycle, but on maintaining the respiratory chain, of which cytochromes are its main component, in good condition to achieve good use of fuels and oxygen.

Porphyrins, components of hemoglobin, myoglobin, and cytochromes, are synthesized from glycine and succinyl-CoA (Fig. 6). The synthesis of a porphyrin molecule consumes eight molecules of succinyl-CoA, and also eight molecules of glycine, two for each pyrrole ring. Porphyrin synthesis depends on the Krebs cycle for the supply of succinyl-CoA in the pyrrole group, derived from porphobilinogen. This synthesis occurs in the cytoplasm. Succinyl-CoA is derived from a-keto-glutarate (intermediate of the Krebs cycle), which is transported there from the mitochondria (Fig. 6).

Porphyrin synthesis is a strictly cataplerotic pathway with a significant net expenditure of succinyl-CoA (8 moles of succinyl-CoA per mole of heme group), a one-way path because the degradation of porphyrins leads to bile pigments, which are excreted. This process decreases the concentration of Krebs cycle intermediates, producing a decrease in their activity. It should be noted that this effect (negative control coefficient on the Krebs cycle flux) does not occur to regulate the activity of the cycle but is imposed by the need to synthesize porphyrins. The daily synthesis of porphyrins and the corresponding spent of succinyl-CoA are shown in Table 1. The consumption of succinyl-CoA, according to the daily turnover of these products, is 10.68 mmol/day (Table 1).

**Table 1.** Daily turnover of heme groups (mmoles/day)

| Protein | Synthesis | Succ-CoA spent |
|---|---|---|
| Mitochondrial cytochromes | 2,26 | 9,04 |
| Hemoglobin | 0,39 | 1,56 |
| Myoglobin | 0,018 | 0,072 |
| Cytochrome P450 | 0,0017 | 0,0068 |
| **Total** | **2,67** | **10,68** |

This means the same cataplerosis of the Krebs cycle, and the same decrease of the main source of energy (ATP), which must be replaced to maintain its activity.

The synthesis of mitochondrial cytochromes consumes 9 mmol of succinyl-CoA/day, and the same of glycine. This means 675 mg of glycine/day, and a total of 802 mg for the synthesis of all the heme groups (Table 1). These cataplerotic syntheses considerably reduce the activity of the Krebs cycle (Fig. 6).

## 4. Physical aging

With advancing age, physical fitness declines occur in skeletal muscle and heart, in the contractile system [25 Nair], in the energy system [25-27], and in collagen [27,28], increasing the likelihood of injury. The invention described here is a method for delaying physical aging.

**Background of nutrient use**

WO 2006/056888 A2 [29] refers exclusively to the use of glycine as a nutritional supplement, with no mention of

proline, lysine, aspartic or citric acid, or vitamin C for this purpose. Glycine intake at doses of 10-20 g/day showed to give good results in repairing physical injuries. The recovery time for injuries described in this document is variable, depending on the type of injury and age. The results we present here demonstrate that the composition we propose (Glycine, L-Proline, L-Lysine, aspartic or citric acid and vitamin C) produce a better and faster recovery.

De Paz-Lugo [8] analyzes the individual effect of glycine, *L*-proline and *L*-lysine on collagen synthesis in chondrocytes cultured in vitro, wherein *L*-isoleucine and *L*-aspartic acid were used as controls. The results show that each amino acid increases collagen production when added to the culture medium, with glycine being the most effective, whose maximum efficacy is maintained from 1.5 mM. The authors state that the increase of these amino acids, especially glycine in the diet, could contribute to fight and prevent osteoarthritis and osteoporosis. However, no analysis is given on the effect of these three amino acids together on the physical form of athletes, and does not make any reference to the object of this application to avoid and repair injuries, and to delay physical aging.

Further, in another study [5] the authors analyzed the control of collagen synthesis in chondrocytes cultured in vitro by glycine, *L*-proline and *L*-lysine in separated experiments, with *L*-isoleucine and *L*-aspartic acid as controls. The results show that proline and lysine exert a positive control (response coefficient) at low concentrations, becoming negative thereafter. No mention to physical exercise is made.

Meléndez-Hevia [30] studied the intake of glycine in the same doses as in the present invention to combat and prevent viral infections. The incidence of influenza and the common cold was investigated in 127 patients (85 treated with glycine, and 42 control patients, treated with *L*-aspartic acid instead of glycine, over a three-year period. In those treated with glycine, the incidence of the viral infections studied disappeared in all patients in the next years, while in the control group the decrease in infections was very low. The intake of aspartic acid was taken as a group control, with no appreciable results.

There are a number of studies that have investigated the effect of nutrients on physical fitness, but they are typically limited to look at the effect of aspartic acid on endurance. Vitamin C is also used, although rather as a placebo. In this way, the art has not been able to obtain significant results for complex functions as we propose here.

It is also known that after administration of K-Mg-aspartate the capacity for prolonged exercise and endurance increases, although some authors have used very high doses in their experiments with rats that cannot be supplemented to humans.

Laborit (1961) [31]. This author made experiments testing the effect of K-Mg-*L*-aspartates in 250 g-rats, that were forced to swim until exhaustion, when they sank and could not again rise to the surface. The first thing to note is that the doses are very high: two doses, each of 125 mg for a 250 g rat. For a 70 kg man, making the allometry corrections [4], it would be 28.35 g, which would have to be taken twice, which is impossible. Here we propose 12 g/day in doses of 3 g spaced according to the duration of the exercise.

Their endurance results between the first test and the second test (spaced two and a half hours apart) are between 15.7% decrease to 24% increase, according to the K/Mg-aspartates product, with or without ATP. However, the main problem of this work is that endurance to exhaustion, involves damage in the contractile system, as we pointed out above. Therefore, this result isolated, is a useless result for the purpose of the work we present here, and for athletes, because endurance increase should not involve any health loss.

Wesson et al. (1988) [32]. These authors have studied the effect of potassium and magnesium salts on endurance until exhaustion. A group of seven athletes ingested 5 g of potassium and 5 g of magnesium aspartate during the 24-hr period prior to the exercise test session. with of the same amount of a sugarless vitamin C powder as placebo. They found that endurance performance was increased by approximately 14%. Thus, they concluded by suggesting that these aspartic acid salts were useful as an ergogenic aid under the conditions found in this experiment. However, the problem is that, as in the previous work [31], exhaustion, involves damage in the contractile system, as we pointed out above. Therefore, this result isolated, is a useless result for the purpose of the work we present here because endurance increase should not involve any health loss.

Maughan et al. (1983) [33]. These authors present contrary results to those of Wesson [32] in a similar trial (6 g of potassium-magnesium aspartate over a 24-h period) in eight athletes of *D*/*L*-aspartic acid mixture (also a small sample) until exhaustion, showing no beneficial effect of oral aspartate administration on work capacity in man and also suggest that the metabolic processes that occur during exercise are not influenced by this treatment. Again, the problem of this work is to test endurance until exhaustion, that involves muscle damage.

Triplett et al. (1990) [34]. As Maughan [33], this work also shows negative results about aspartic acid in nine boys. Administration of 5 g of K-asp, 5 g Mg-asp, and 1 g vit C (as placebo) 12 hours before the exercise session gave no significant differences between placebo and aspartate. Thus, they conclude that aspartates are not effective in reducing fatigue.

Ahlborg et al. (1968) [35]. The effect of K-Mg-aspartate on the capacity for continuous prolonged standardized physical exercise (exercise times about 90 min) was investigated in six normal young men. After administration of potassium-magnesium-aspartate (1.75 g of *L*-aspartate every 6 hours), the capacity for prolonged exercise increased about 50 per cent. The problem is again, the little or no meaning of measuring the effect on endurance to exhaustion as an

isolated variable, because it involves muscle damage.

Gupta & Srivastava (1973) [36]. These authors used the same protocol as Ahlborg [35]. (K-Mg-aspartate) in five men 20 to 25 years old exercising to exhaustion on a bicycle ergometer. Their results showed an increase of endurance of 18.2%. Again, increase of endurance to exhaustion involves muscle damage, which does no meaning on physical fitness for the purpose of the work we consider here.

Barnes et. al. (1964) [37]. These authors have investigated the effect of aspartic acid and its potassium and magnesium salts on the increase in blood ammonia that occurs during prolonged exercise until g exhaustion. They made their experiments with rats by forcing them to swim to exhaustion, as detailed in [31]. Two doses of 100 mg/100 g of body mass spaced 17 hours apart; the last one, one hour before the forced swimming exercise were administered. Their results show that this amino acid prolongs the time until physical exhaustion by 43%-50%. These authors use very high doses (the same as we have pointed out in [31]), which if applied to a 70 kg man, taking into account the allometry [4], would be two doses of 56,7 g spaced 17 hours apart, which is again impossible. What we propose here is 12 g/day in doses of 3 spaced according to the duration of the exercise. However, the main problem is again that increase of endurance to exhaustion involves muscle damage, which does no meaning on physical fitness as we consider here.

It is known in the art that the intake of *L*-aspartic acid promotes fat consumption, but it is aimed at fighting a series of health conditions other than those of the present invention (obesity, diabetes, hypertension, and anemia, among others).

Meléndez-Hevia, E. & Meléndez-Morales, D. (2006) *L*-aspartic acid for the treatment of problems in connection with the fat or glucose metabolism. WO 2006/087232 A1 [38]. In this document the intake of *L*-aspartic acid is proposed to promote fat consumption, aimed at fighting a series of health problems (obesity, diabetes, hypertension, and anemia, among others) different than those we propose here, and there is no reference to increasing physical fitness in any of its aspects discussed here, nor to curing or avoiding injuries, or to fight sports aging. In the present application we propose a combination of ingredients, all of them working coordinately for the same purpose.
problems (obesity, hypertension, diabetes, etc.), because they cease to exist with the treatment. We propose, therefore, that the treatment described to improve physical condition has a very rapid effect, but that it must be prolonged to maintain fitness.

## References

1. Dvorak J. & Junge, A. (2000) Football Injuries and Physical Symptoms. American Journal of Sports Medicine. 28(5 suppl), 3-9.

2. Robi, K., Jakob, N., Matevz, K. et al. (2013) The physiology of sports injuries and repair processes. Current Issues in Sports and Exercise Medicine.

3. Baoge, L., Van Den Steen, E., Rimbaut, S. et al. (2012) Treatment of skeletal muscle injury: A review. International Scholarly Research Network ISRN Orthopedics, 2012, ID 689012.

4. Meléndez-Hevia, E., de Paz-Lugo, P., Cornish-Bowden, A. & Cárdenas, M. L., (2009) A weak link in metabolism: the metabolic capacity for glycine biosynthesis does not satisfy the need for collagen synthesis. Journal of Biosciences, 34, 853-872.

5. de Paz-Lugo, P., Lupiáñez, J. A., Sicilia, J. & Meléndez-Hevia, E. (2023) Control Analysis of collagen_synthesis by glycine, proline and lysine in bovine chondrocytes in vitro. - Its relevance for medicine and nutrition. BioSystems, 232, 105004.

6. Cochella, L., Green, R., 2005. Fidelity in protein synthesis. Curr. Biol. 15, R536-R540.

7. Meléndez-Hevia, E. & de Paz-Lugo, P. (2008) Branch-point stoichiometry can generate weak links in metabolism: The case of glycine biosynthesis. Journal of Biosciences, 33, 771-780.

8. de Paz-Lugo, P., Lupiáñez, J. A. & Meléndez-Hevia, E. (2018) High glycine concentration increases collagen synthesis by articular chondrocytes in vitro: acute glycine deficiency could be an important cause of osteoarthritis. Amino Acids, 50, 1357-1365.

9. Wu, G., Bazer, F. W., Burghardt, R. C. et al. (2011) Proline and hydroxyproline metabolism: implications for animal

and human nutrition. Amino Acids. 40, 1053-1063.

10. Miller, B.F., Olesen, J.L., Hansen, L., et al. (2005) Coordinated collagen and muscle protein synthesis in human patella tendon and quadriceps muscle after exercise. J Physiol. 567(pt 3), 1021-1033.

11. Miller, B. F., Hansen, M., Olesen, J. L. et al. (2007) Tendon collagen synthesis at rest and after exercise in women. J Appl Physiol 102, 541-546.

12. Kjaer, M., Jorgensen, N. R., Heinemeier, K. & Magnusson, S. P. (2015) Exercise and regulation of bone and collagen tissue biology. Progress in Molecular Biology and Translational Science, 135, 259-291.

13. Fatouros, I. G., & Jamurtas, A. Z. (2016). Insights into the molecular etiology of exercise-induced inflammation: opportunities for optimizing performance. Journal of Inflammation Research, 9, 175-186.

14. Olson K, Zimka O, Pasiorowski A, et al. (2018) Tiredness, fatigue, and exhaustion as perceived by recreational marathon runners. Qualitative Health Research, 28, 1997-2010.

15. Bremel, R., Weber, A. (1972) Cooperation within Actin Filament in Vertebrate Skeletal Muscle. Nature New Biology, 238, 97-101.

16. Maruyama, K. & Weber, A. (1972) Binding of adenosine triphosphate to myofibrils during contraction and relaxation. Biochemistry, 11, 2990-2998.

17. Weber, A. (1999) Actin binding proteins that change extent and rate of actin monomer-polymer distribution by different mechanisms. Molecular and Cellular Biochemistry 190, 67-74.

18. Pemrick, S. & Weber, A. (1976) Mechanism of inhibition of relaxation by N-ethylmaleimide treatment of myosin. Biochemistry, 15, 5193-5198.

19. Fridén, J. (1984) Changes in human skeletal muscle induced by long-term eccentric exercise. Cell Tissue Res (1984) 236:365 372

20. Appell, HJ., Soares, J.M.C. & Duarte, J.A.R. (1992) Exercise, muscle damage and fatigue. Sports Medicine 13, 108-115

21. McHugh, M. P., Connolly, Connoly, D. A. J., Eston, R. G., et al. (1999) The role of passive muscle stiffness in symptoms of exercise-induced muscle damage. American Journal of Sports Medicine, 27, 594-599.

22. Fitts, R. H. (2008) The cross-bridge cycle and skeletal muscle fatigue. J Appl Physiol 104: 551-558.

23. Brownstein, C. G., Dent, J. P., Parker, P., et al. (2017) Etiology and recovery of neuromuscular fatigue following competitive soccer match-play. Frontiers in Physiology, 8, 1-14.

24. Constantin-Teodosiu, D. & Constantin, D. (2021) Molecular mechanisms of muscle fatigue. Int. J. Mol. Sci. 22, 11587.

25. Nair, K. S. (2005) Aging muscle Am. J. Clin. Nutr. 81, 953-963.

26. Figueiredo, P. A., Mota, M. P., Appell, H. J. & Duarte, J. A. (2008) The role of mitochondria in aging of skeletal muscle. Biogerontology, 9, 67-84.

27. Kwak, H.-B. (2013) Aging, exercise, and extracellular matrix in the heart. Journal of Exercise Rehabilitation, 9, 338-347.

28. Gumpenberger, M., Wessner, B., Graf, A., et al. (2020) Remodeling the skeletal muscle extracellular matrix in older age-effects of acute exercise stimuli on gene expression. International Journal of Molecular Sciences, 21, 7089.

29. Meléndez Hevia, E. (Inventor): Glycine as a diet supplement for the treatment of a wide range of health problems

that result from underlying metabolic disorders. International Publication Number: WO 2006/056888 A2. Priority Data: 60/599,908; 9 August 2004 (09.08.2004). International Filling date: 8 August 2005. International Publication date: 1 June 2006.

30. Meléndez-Hevia, E., de Paz-Lugo, P. & Sánchez, G. (2021) Glycine can prevent and fight virus invasiveness by reinforcing the extracellular matrix. Journal of Functional Foods, 76, 104318.

31. Laborit, H. (1961) Potassium aspartate and magnesium aspartate fatigue recovery promoting process and compositions. United States Patent Office 3,009,859 - patented Nov. 21, 1961.

32. Wesson, M., Mcnaughton, L., Davies, P., & Davies, P. (1988). Effects of oral administration of aspartic acid salts on the endurance capacity of trained athletes. Research Quarterly for Exercise and Sport, 59, 234-239.

33. Maughan, R. J. & Sadler, D. J. M. (1983) The effects of oral administration of salts of aspartic acid on the metabolic response to prolonged exhausting exercise in man. Int J Sports Med, 4, 119-123.

34. Triplett, N. Travis; Stone, Michael H.; Adams, Chip; Allran, Kevin D.; Smith, Tim W. (1990). Effects of aspartic acid salts on fatigue parameters during weight training exercise and recovery. Journal of Applied Sport Science Research, 4, 141-147.

35. Ahlborg, B., Ekelund, L.G. and C.G. Nilsson, C. G. (1968) Effect of potassium-magnesium-aspartate on the capacity for prolonged exercise in man. Acta Physiol. Scand. 74, 238-245.

36. Gupta, J. S. & Srivastava, K. K. (1973) Effect of potassium-magnesium aspartate on endurance work in man. Indian J. Exp. Biol. 11, 392-394

37. Barnes, R. H., Alcazar Labadan, B., Siyamoglu, B. & Robert B. Bradfield, R. B. (1964). Effects of exercise and administration of aspartic acid on blood ammonia in the rat. Am. J. Physiol. 207, 1242-1246.

DESCRIPTION OF THE INVENTION

The present invention is a combination of glycine, *L*-proline, *L*-lysine, *L*-aspartic acid (or citric acid, or their $K^+$-$Mg^{2+}$ salts), and vitamin C, to improve physical fitness preventing and repairing physical injuries, fatigue, and physical aging.

The combination of the invention cannot be simplified by testing the effect of each component separately, because physical fitness is a complex function that includes five variables: namely, strength, velocity, achievements, endurance, and lack of discomfort during performance. Recognizing the additional uses for the combination of the nutrients glycine, *L*-proline, *L*-lysine, *L*-aspartic acid (or *L*-aspartate), citric acid (or citrate) and vitamin C (*L*-ascorbic acid or *L*-ascorbate) the present invention is directed to exploiting this opportunity and thereby contributing new treatment method for a wide range of health conditions related with physical exercise.

In a preferred embodiment, the present invention is a method to improve physical fitness and treat or prevent related health conditions that are aggravated, such as exhaustion, fatigue, physical aging, and injuries. More preferably, this method includes the daily ingestion of a therapeutically effective dosage of the combination or mixture of nutrients, even more preferably in crystallized powder dissolved in water, yogurt or fruit juice at the rate of (g/day): glycine 5.0-20.0; *L* -proline 5.0-10.0; *L* -lysine 2.5-5.0; *L*-aspartic, or citric acid (or their $K^+$-$Mg^{2+}$ salts) 6.0-18.0; vitamin C 1.0-2.0.

In an even more preferable embodiment, the diet contains an amount of protein of 1.0-1.5 g/kg body mass daily.

Yet another preferred embodiment of the present invention is the supply of the mixture described above in other galenic forms such as drinks, or creams or gels for topical application.

A result of the intake of the combination of the present invention is the very short time it takes to take effect to improve physical fitness: 58%, and 38% in athletes under and over 35 years of age, respectively, in three weeks, which continues to rise to 70% and 47%, respectively, as the treatment is prolonged. Disease like obesity, hypertension, diabetes, etc. cease to exist with the treatment in a certain time.

The general object of the present invention is to provide a dietary supplement to improve physical condition (fitness) in four aspects of sports practice, and physical work, in general, which are:

1. To increase physical-sports performance (performance), which includes strength, velocity, effectiveness (achievements), increase endurance without tiredness, and avoid joint and muscle discomfort (which means injuries) during and after exercise.

2. To reduce the time of fatigue for full recovery after a match, so that the athlete is in optimal condition to face the next one in the shortest possible time.

3. To prevent, reduce, and cure physical injuries that often occur in sports practice and, in general, in any physical work.

4. To increase physical and sports-related youth, in order to prolong the active life of competitive athletes, and in general of all those whose work depends on their physical activity.

The present invention described as a nutritional supplement is capable of being practiced in various ways, in a variety of products, such as an energy drink, creams or gels for topical application, etc. Likewise, the present invention can be applied to animals -especially, but not limited to- sports animals, such as racing horses or dogs, hunting dogs, beasts of burden, physical exhibition animals, such as fighting bulls, bullfighting horses, etc.

It is a very simple means of treating the aforementioned conditions in humans and animals. It should be noted that, apart from the health and well-being of the individual, all of this has a great economic interest, especially in professional athletes, and in jobs that are highly dependent on physical fitness.

**Physical fitness and performance**

In the present invention, it is considered the physical condition of each athlete, omitting the specific technique of each type of exercise, and the teamwork (for sports that have it), which is necessary for each individual task. These two characteristics are taken for granted, limiting the present invention to the physical conditions of each individual.

Physical fitness includes five variables, namely strength (s), velocity (v), achievements-effectiveness (a), endurance (e), and discomfort (d). The aim to get maximal fitness is maximizing strength, velocity, and endurance, and minimizing physical discomfort or pain.

The nutrients of the invention work coordinately on each of these variables, not simply the sum of the effect of each nutrient separately, So if one or several of them are omitted, the function can give a poor or incomplete result.

To check the effect of the treatment, each of these variables is evaluated on a scale of 1 to 10, giving a value of 5 to all at the beginning, and taking their value at regular intervals of time (for example, every week).

Taking this into account, the inventors use a global fitness function (F) for presenting results that includes all five, as:

$$F = \frac{Mean\ \ (s, v, a, e)}{d} \qquad \text{(Eqn 1)}$$

So, if, for example, a large increase in speed brought with it a large decrease in effectiveness, its effects would be cancelled out. In the same way, the useful endurance time for an athlete is not a variable that has meaning in isolation, because the objective is not about withstand exercise for a long time, but to be in optimal condition during that time to perform in strength (s), velocity (v), achievements-effectiveness (a), and minimizing the discomfort (d).

Thus, if a large increase in endurance is achieved based on important decreases in strength, velocity, or achievements, or an important increase of discomfort the value of the function F could be very low. In endurance tests where only the time until exhaustion is measured, obviously strength, speed and effectiveness are lost, and discomfort takes a high value by forcing the machine excessively. Therefore, previous published works on any of these variables isolated, i.e., endurance without considering the lost or decay of the others [31-37,] do not contribute to the present invention.

In addition, to the variables considered in Eqn (1), it is equally important: (a) to reduce fatigue after the match, to be ready for the next as soon as possible; (b) to avoid injuries or to achieve their healing in the shortest time possible; and (c)

to fight physical-sports aging, in order to prolong the active life of competitive athletes, and in general of all those whose work depends on their physical activity, such as acrobats, film extras, police officers, firefighters, etc.

With the exception of injuries, that are discussed below, the other conditions as mentioned have always been related to the expenditure and production of ATP, so that there is the necessary amount for the intensity of physical exercise required for a given job. This is certainly important, but the issue offers more concerns than it may seem at first glance. To introduce all of them, special attention should be paid to three aspects: (1) the energy flux, (2) the mechanism of the contractile system, and (3) the reinforcement of the passive mechanical system (collagen and elastin).

**Energy flux - ATP expenditure and replacement**

As energy availability is behind most performance variables, most -if not all- studies in this field refer to the production and consumption of ATP, in order to maintain its concentration constant. Relevant is that it is not merely an energy requirement, but a kinetic need for the contraction cycle. The ATP consumed in any metabolic process must be replaced at the same speed. This issue mainly concerns physical exercise, which is the main spent of ATP. During a match, there is a large flux of ATP expenditure by the contractile apparatus. It is common, and always foreseeable that the need for ATP expenditure is greater than its production. So, given that physical exercise is a priority, whether for the prey to escape from the predator, or for the predator to hunt it, the muscle has the creatine-phosphate system, which is not a 'type of anaerobic metabolism', but a buffer system to keep the ATP concentration constant; in extreme cases, its capacity is very short (»3 seconds), but sufficient to get out of a tight spot. But if this time is prolonged, then the concentration of ATP decreases, and this disturbs the contraction cycles, by slowing down the release of the rigor bridges.

ATP hydrolysis in the myosin reaction ATP + $H_2O$ ® Myo-ADP + Pi depends little on the concentration of ATP. Its concentration in muscle cytoplasm is very high compared to other cells (1.0-2.0 mM), whereas the KM of myosin ATPase is very low (8 mM). With these kinetic properties, myosin ATPase is »100% saturated at 1.5 mM-ATP. A high decrease in ATP concentration (say to, 0.5 mM) would give a saturation 98%. Thus, the decrease in force cannot be explained at all by a decrease in ATP for myosin ATPase. These features demonstrate the priority that natural selection has given to the work of the muscle, to escape from danger, or to attack prey.

However, maintaining a high ATP concentration is necessary for another part of the contraction mechanism, not specifically for the ATPase reaction, but to dissociate the ADP product from the enzyme by Myo-ADP + ATP ® Myo-ATP + ADP

The Krebs cycle

The sources of acetyl-CoA (the real substrate of the Krebs cycle) are abundant because there is always a large reserve of fatty acids, and enough glycogen in the liver to support prolonged exercise. Thus, the cause of poor physical performance could be more in the activity of the Krebs cycle to degrade acetyl-CoA at the speed imposed by the need for ATP in the practice of a sport. This could be done by increasing the activity of the enzymes, but it is difficult to achieve an appreciable effect, since they are highly regulated and very interconnected with other pathways. The strategy of the present invention is to increase the catalytic capacity of the Krebs cycle by enhancing anaplerosis.

Let us assume that all the enzymes in the cycle operate at their maximum regulatory activity, but always dependent on the concentration of their particular substrates, with about 25-50% saturation. Then, it is still possible to increase the activity of the cycle by another little-explored procedure, which consists of increasing its catalytic capacity.

The rate of an isolated reaction (Scheme 1) with respect to the substrate concentration can be hyperbolic (Michaelis-Menten mechanism), sigmoid (allosteric enzymes with positive cooperativity), etc. In all cases, the rate is linearly proportional to the enzyme concentration, regardless of the kinetic mechanism.

**Scheme 1**

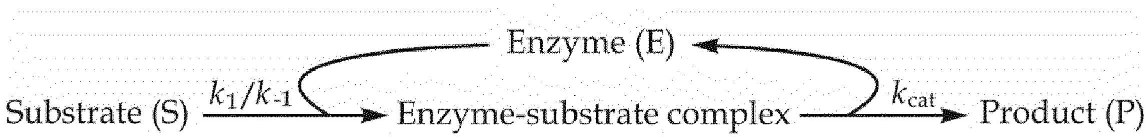


The Krebs-cycle pathway (Fig. 1) starts with the condensation of acetyl-CoA (AcCoA) and oxaloacetate (OAA). However, a distinction must be made between these two. It can be briefly represented as a single reaction encompassing the entire sequence as a black box, as shown in Scheme 2:

## Scheme 2

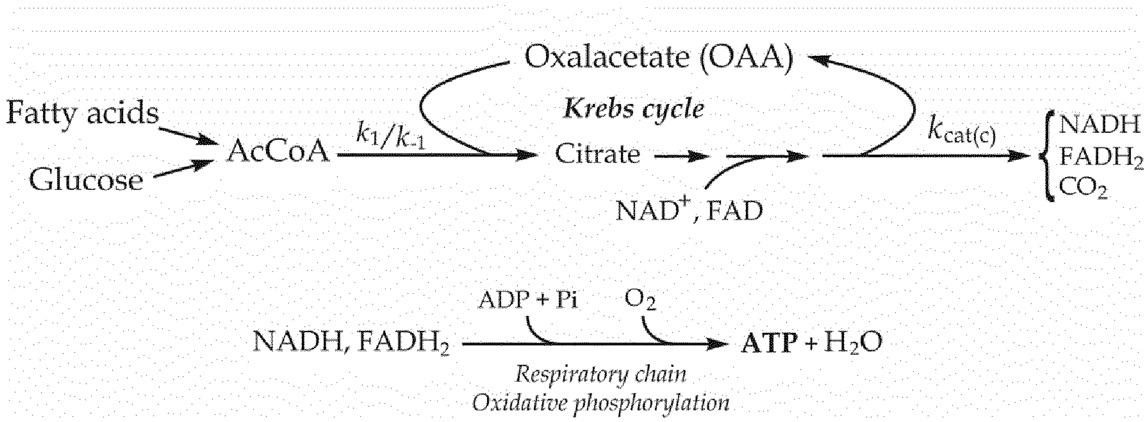


Comparing this with the previous one, oxaloacetate is not a mere substrate but a feeder (catalyst) for the oxidation of acetyl-CoA, and the same can be said of any carbon intermediate, since all of them end up being converted into OAA. This fact was noted by Albert Szent-Györgyi in 1935 when he observed that the addition of a C4 dicarboxylic acid (succinate, fumarate or malate) or a C6 tricarboxylic acid (citrate or isocitrate) increased the reaction rate to a degree that could not be explained merely by the oxidation of the added product. In 1937, Hans Krebs duly interpreted this fact, proposing the cyclic pathway that bears his name (Fig. 1).

Therefore, an increase in the concentration of OAA (or any carbon intermediate) produced by an eternal source will cause a catalytic increase in the rate of the cycle, and the opposite will occur with a decrease in its concentration when intermediates are diverted to other pathways.

These effects are not proportionally linear as in the case of an isolated enzyme, discussed above; in fact, the increase factor can be equal to, or greater than 1, according to the kinetic mechanisms (Michaelis, allosteric, etc.), and regulatory effects operating on the enzymes of the cycle, but they will be greater than an equal change in the AcCoA substrate concentration, provided that the enzymes are not saturated, which is the case.

Since increasing the concentration of any intermediate of the cycle should produce a catalytic effect similar to that of OAA, the inventors have tested the effects of a wide range of OAA precursors, either internal to the cycle or external to it, all of which are capable of being converted to OAA by different metabolic reactions. These tests were made with laboratory experiments on mitochondria isolated from rat diaphragm and pigeon pectoral muscle, both aerobic red muscles. The system was developed in an oxygen electrode, to measure the activity of the respiratory chain by oxygen consumption, in the presence of palmitate (C16) as an oxidizable non-anaplerotic substrate, ADP, phosphate, and a cycle feeder. This experimental system is a modification of the classic one (Fig. 7A) described by Chance et al. to demonstrate the oxidation of any product by any route coupled to phosphorylation.

The system presented here (Fig 7B) has abundant ADP and a limiting feeder concentration, so that the reaction responds to increasing feeder concentration.

The results, shown in Figure 8 and Table 2, with palmitoyl-carnitine as a precursor substrate of acetyl-CoA, showed that the most effective feeders (or precursors of them, or anaplerotic products) to increase the activity of the Krebs cycle are *L*-aspartic acid (or *L*-aspartate) and citric acid (or citrate).

**Table 2.** Degree of activation. [State B/State A in Figure 7(B)] of the most effective feeders, or their precursors, of the Krebs cycle in mitochondria isolated from rat diaphragm and pigeon pectoral muscle, with 20 mM palmitoyl-carnitine as substrate; see Figures 7(B) and 8.

State B/State A

| Control | | Rat | | Pigeon | |
|---|---|---|---|---|---|
| Aspartate | Citrate | Aspartate | Citrate | Aspartate | Citrate |
| 13,45 | 5,431 | 60,00 | 48,217 | 26,49 | 2,256 |

### The contractile apparatus

The mechanism of contraction is based on the interaction of the thick filaments (myosin) with the thin filaments (actin) of the sarcomere (contractile unit of the muscle). In a resting state, these interactions are not possible because the actin filaments are 'shielded' by tropomyosin.

The release of calcium from the cisternae of the sarcoplasmic reticulum into the cytoplasm, as a consequence of the nervous stimulus, unblocks this barrier, making the interaction of the myosin heads with the actin filaments possible.
Sarcomere contraction involves between 80 and 150 cycles, depending on the muscle. The kinetic mechanism of a cycle is shown in Scheme 1. After a cycle of contraction by ATPase reaction, the myosin of the contracted bridges remains bound to ADP, which dissociates very slowly. A fraction of them dissociates, it being ready for the next cycle, while another fraction remains bound to actin, forming rigor bridges. These bridges hold the actin filament, preventing it from recoiling by the spring effect of titin, so that the free (relaxed) myosin heads bind to actin at more distant points. When the rigor bridges eventually relax, the new heads contract and the second cycle occurs, and so on.

## Injuries of the contractile system

Figure 9 shows a model of contraction (half a sarcomere and five myosin heads on the thick filament, for simplicity), which answers the questions raised in the previous section (Fig. 4).

## Normal cycles with high ATP concentration

On the left (Fig. 9A) the mechanism is represented with the normal physiological high concentration of ATP (1.0-2.0 mM). Under these conditions, the mechanism works perfectly, without problem, with the myosin ATPase saturated at 100%, producing all the contraction cycles that are necessary (between 80 and 150, depending on the muscle).
Each cycle starts with the binding of the relaxed myosin heads (empty ellipses) to the thin filament actin when they are within reach (m1, m2, m3 in the State a; *m1, m2, m4* in State ***f***, etc.); this binding activates the myosin ATPase (black ellipses) of the same heads); ATPase binds to ATP, and the reaction ATP + $H_2O$ ® ADP + Pi occurs, which produces a »60° turn of the head, with a displacement of the actin filament of »18 nm in the half sarcomere (»36 nm in the complete sarcomere).
Then (State e) the products (ADP and Pi) are released from some heads (*m1, m2*), remaining relaxed ready for the next cycle, but others (*m3*) remain bound to ADP and actin (grey ellipses marked with R) forming rigor bridges.
The function of these rigor bridges is crucial, as they hold the displaced filaments, preventing them from moving back due to the tension of the titin spring, when the myosin heads that caused the displacement relax (*m1, m2* in state ***e***; *m1, m3* in state ***i***, etc.).
This allows the relaxed myosin heads (*m1, m2,* in state *e*) to be added in the following cycle, with others being added that could not do so in the previous cycle (m4). The actin filament is held in place by rigor bridges, which allow these heads (*m1, m2, m4* in State ***f***; *m1, m3, m5* in state ***j***) to interact with the actin filament at points closer to the Z disk.
Finally, all rigor bridges end up dissociating, remaining as relaxed heads (*m3* in state ***g****; m2, m4* in State ***k***, etc.), ready for the next cycle, as in State ***a***, so that under normal conditions (high ATP concentration), they are transient rigor bridges.
The different dissociation of ATPase-ADP to give relaxed heads or rigor bridges (for example, the steps of the m1, m2, m3 heads, from State ***d*** to State ***f***) is achieved because the dissociation of the ATPase from ADP is slower than the binding of the free ATPase to ATP [18].
This dissociation is stochastic, and it is not possible to determine which heads will remain free (relaxed) and which will remain as temporary rigor bridges. Everything happens at high speed, and the process achieves its deterministic objective (the advance of the actin filaments in the next cycle) because each myosin filament has 300 myosin heads in each half sarcomere, so there will always be a certain fraction of temporary rigor bridges (which will hold the advanced actin filament to prevent it from moving backwards) and a fraction of relaxed heads to interact with the actin filament, which will make the advance at points closer to the Z disk.

## Problem with low ATP concentration

Figure 9(B), on the right, represents the contraction with low ATP concentration. Unlike what is seen in the normal situation (A), if the ATP concentration drops too low, to »0.5 mM, or lower, then problems appear, but not due to a decrease in the activity of the ATPase, but rather to the dissociation of the rigor bridges. This dissociation, at the speed necessary for the normal cycle, requires a high concentration of ATP, because if it drops too low, the dissociation of the rigor bridges slows down, causing some to remain (resistant) (*m3* in states ***g-l***; *m1* in States ***j-l***) when the heads arranged for this contract (*m1, m2, m4* in State *g*; *m2, m4, m5* in State *k*).
Under these conditions, contraction (ATPase activity) can occur without major differences, since the low $K_M$ of myosin due to ATP makes it reach a saturation of 98% with 0.5 mM of ATP, but it requires greater effort because it not only has to overcome the macroscopic resistance of the normal obstacle, but also the microscopic resistance of the obstacle of the rigor bridges.
Since the actin filament is held by the rigor bridges, the forced contractions deform the rigor bridges making them

rigid, resistant and persistent (vertical grey ellipses marked with R) [18] causing the structure of the sarcomere to deteriorate, which are microscopic lesions, felt by the athlete as 'tiredness'.

Therefore, tiredness is not simply a lack of ATP for the activity of the myosin ATPase, since this is not noticeably reduced by a decrease in ATP, even if it is very large, but it is the warning of a muscular damage that hinders or even prevents the functioning of the cycle due to the poor dissociation of the rigor bridges.

Thus, under a low ATP concentration, the dissociation of the rigor cross bridges decreases, and contraction becomes slower and more difficult although ATP concentration were enough for ATPase activity. This leads to a significant decrease in the capacity of contraction, which is the real cause of the loss of physical performance, tiredness, and even injuries.

Let us consider, for example, about aiming the ball. If the player is tired, although cerebellar coordination works well, the shortage of ATP can disable a series of fibers (cells) due to an excess of rigor bridges without dissociating. Then, the system will not have all the cells willing to respond to the nervous stimulus, and the complete response of that coordination will not be possible, which explains the loss of aim inherent to tiredness.

This distortion is really a pathology, however slight and transitory it may be, since it prevents the normal functioning of the contractile apparatus in several ways: the result achieved has a high probability of being ineffective because it will not have sufficient force; it will not have the desired speed; and it will have lost its aim.

Excessive, unresolved bridging of tired muscle obviously affects all aspects of fitness, causing lack of strength, decreased speed, poor effectiveness, and poor endurance.

In these conditions, the athletes should notice their strength diminished, and even pain [e.g., 13], which is a warning that there is a dangerous deficit of ATP, and they should stop the effort. In this condition, the player must be substituted, when possible,

But if they persist in this activity, the abnormal situation of the contractile apparatus will deteriorate more and more, because the fraction of rigor bridges that are not resolved will be greater and greater; the contraction will become increasingly difficult, and if the condition persists, it may lead to collapse, including the cardiac fibers, which explains the heart attack with death, which unfortunately occurs all too frequently in long and medium-duration sports (running, football, racing, and even basketball). The fact that this situation can lead to death demonstrates that it is a pathology, or a warning of a pathology, which can be greatly aggravated by continuing to exercise under such conditions.

The increase in rigor bridges can be aggravated if the player insists on maintaining his activity with a shortage of ATP, which is noticed by the loss of strength, speed, and effectiveness. This massive increase in rigor bridges causes serious damage to the muscles, which become useless for exercise. This is the cause of fatigue, which can require several days to recover until all the myosin heads are relaxed.

Anyway, the best way to avoid it is to enhance the ATP synthesis by increasing the anaplerosis of the Krebs cycle, as described in this invention.

**Strength of the passive mechanical system**

Apart from the contractile apparatus, good physical fitness also depends on the strength of the passive mechanical system (bones, cartilage, tendons, ligaments, and in general the entire extracellular matrix) whose main components are collagen and elastin. These proteins are a main basis of physical injuries and two aspects must be considered: their repair (which affects everyone), and their frequency, which is fortuitous in non-athletes, but very high in professional athletes.

Many times, repair is not fully achieved; many professional athletes, after weeks or months (even years) of inactivity due to an injury, return to the court, perhaps not very resentful, but not 'at 100%'. By continuing to feel certain discomfort, they play with fear; this greatly reduces the natural risk implicit in the plays, which their work would require. Most high-level athletes recognize that they often play with physical discomfort; the problem solved by the invention is to achieve good physical fitness in all aspects.

Collagen and elastin age due to poor turnover because their synthesis is very complex, with a very high degree of error, which means a high waste of material and energy making injuries more likely. Thus, improving their biosynthesis can greatly help to improve the physical performance of athletes, as well as prevent and resolve injuries.

**Hydroxylations in collagen synthesis and the Krebs cycle**

Correct collagen synthesis (Fig. 2) involves the synthesis of tropocollagen, and the hydroxylations of proline and lysine. These hydroxylations occur in cytoplasmic vesicles. The synthesis of a-keto-glutarate (aKG), co-substrate of hydroxylases, occurs mainly from the Krebs cycle.

As hydroxylations yield succinate as a product, which is reincorporated into the Krebs cycle (Fig. 5), they are not a strictly cataplerotic process, but a bypass of the cycle, omitting the succinyl-CoA synthetase step; this means damage to porphyrin synthesis and a 25% decrease in reduction equivalents (NADH) with the respective consequences in ATP

synthesis.

Therefore, improving collagen synthesis by reducing the extent of the tropocollagen cycle will not only increase the strength of the mechanical system, but will also do so for the energy function of the Krebs cycle. This must be corrected to ensure the supply of ATP, especially for physical exercise.

**Proline requirement**

Although proline is considered a non-essential amino acid, as the human metabolism can synthesize it, its capacity for biosynthesis must have a limit, making it conditionally essential. Regarding that explained above, the cause of this need should be the procollagen cycle, whose low efficiency consumes a large amount of it.

Hydroxylations are catalyzed by proline hydroxylase, which uses oxygen and a-keto-glutarate, and vitamin C as substrates.

These hydroxylations are very complex, since only a certain percentage of the prolines (9.1% of collagen residues) must be hydroxylated, leaving the rest (13%) as simple proline, and they must be carried out in specific proline residues, not in any of them.

From the calculations presented in Table 3 the cause of the deficit can be a hydroxylation defect, about 5-10%; if more, the proline deficiency caused would be impossible to bear.

The defect in the number of hydroxylations, reduced to »5% of those due), causes a deficit of »8g of proline/day. However, it should be noted that, even without hydroxylations (first row of Table 3), there is already a proline deficit of »4 g/day.

**Table 3.** Proline consumption in the tropocollagen cycle according to the percentage of hydroxylation. The non-hydroxylated proline residues (13% in the correct collagen) are mostly recycled (95%), but the hydroxylated residues (9.1% in the correct collagen) are not recycled, since their degradation yields other products. Therefore, an extra supply is required in the procollagen cycle to recover the hydroxylated proline.

**Table 3.** Need of proline, according to the degree of hydroxylation

| | Amount of proline necessary (g/day) | | | |
|---|---|---|---|---|
| % HO-Proline | Pro Simple | HO-Pro | Total | Deficit* |
| 0 | 11.75 | 0,00 | 11.75 | 3.75 |
| 5 | 11.51 | 4.70 | 16.21 | 8.21 |
| 10 | 11.25 | 9.68 | 20.93 | 13.00 |
| 25 | 10.55 | 24.20 | 34.75 | 26.75 |
| 50 | 9.35 | 48.35 | 57.70 | 49.70 |
| 75 | 8.12 | 72.60 | 80.72 | 72.72 |
| 100 | 6.90 | 104.00 | 104.00 | 96.00 |

* Deficit = Total Proline necessary - [Synthesis + diet (8 g)]

From the calculations presented in Table 3 the cause of the proline deficit can be a hydroxylation defect, about 5-10%; if more, the proline deficiency caused would be impossible to bear.

The defect in the number of hydroxylations, reduced to »5% of those due), causes a deficit of »8g of proline/day. However, it should be noted that, even without hydroxylations (first row of Table 3), there is already a proline deficit of »4 g/day.

**Lysine requirement**

Although lysine is not a particularly abundant amino acid in collagen (2.7% of residues), 50% of them are also hydroxylated, which is essential for the formation of the triple helix. In the tropocollagen cycle, all hydroxylated lysine residues are therefore lost. Table 4 shows that no more than 10% hydroxylations are possible; it is therefore necessary to increase lysine intake as much as possible to avoid its loss.

**Table 4.** Lysine consumption in the tropocollagen cycle according to the percentage of hydroxylation. The non-hydroxylated lysine residues are mostly recycled (95%), but the hydroxylated residues are not recycled, as their degradation yields other products. Therefore, an extra supply of all hydroxylated lysine is required in the procollagen cycle.

**Table 4.** Need of lysine, according to the degree of hydroxylation

| | Amount of lysine necessary (g/day) | | | |
|---|---|---|---|---|
| % HO-Lysine | Lys simple | HO-Lys | Total | Deficit* |
| 0 | 1,82 | 0 | 1,82 | No |
| 5 | 1,77 | 0,91 | 2,68 | No |
| 10 | 1,73 | 1,82 | 3,55 | No |
| 25 | 1,59 | 4,56 | 6,15 | 1-2 |
| 50 | 1,36 | 9,12 | 10,48 | 5-6 |
| 75 | 1,14 | 13,68 | 14,82 | 10-11 |
| 100 | 0,91 | 18,23 | 19,14 | 14-15 |

* Deficit = Total Lysine necessary minus in the diet (4-5 g/day)

Calculations in Table 4 show that no more than 10% hydroxylations are possible; it is therefore necessary to increase lysine intake as much as possible to avoid its loss.

Therefore, improving collagen synthesis by reducing the extent of the tropocollagen cycle will not only increase the strength of the mechanical system, but will also do so for the energy function of the Krebs cycle. This must be corrected to ensure the supply of ATP, especially for physical exercise.

On the other hand, physical exercise stimulates collagen synthesis [10-12], which again makes it necessary to supplement all the products mentioned in the composition proposed by the inventors.

**Elastin**

Elastin is the protein mainly responsible for the elasticity of tissues (muscle, heart, skin, arteries, etc.). Its synthesis is equally complex, like that of collagen, with the same waste, the consequent recycling and the expenditure of substrates. Although it is less abundant than collagen, its need for substrate use is the same, since it depends on aa-tRNAs (Gly, Pro, Lys, etc.).

Therefore, the deficiency of glycine, proline and lysine seen above for collagen synthesis also affects the synthesis of elastin, whose deficiency will decrease the elasticity of the tissues, an indispensable condition for physical exercise and for many other properties of the body, such as the skin on the joints, or the mechanical activity of the arteries and the heart.

**Porphyrin synthesis**

Maintenance of the ATP factory involves a high porphyrin synthesis Table 1), which spends a large amount of glycine, and the same of succinyl-CoA in a cataplerotic pathway (Fig. 6), considerably reducing the activity of the Krebs cycle.

These precursors (glycine and a feeder) must be replaced. The feeder can be supplied as explained above; as for glycine, the more direct, strategy is to directly supply the necessary amount, adding it as a food supplement to the diet of athletes. All this is included in the combination of nutrients of this invention.

**Fatigue**

The low ATP contraction mechanism shown in Figure 9(B) also explains the cause of persistent fatigue over several days. Fatigue is the consequence of excessive physical effort that has produced many rigor-resistant bridges, by forcing contraction at low ATP concentration, with the consequent deformities of the sarcomere structure, all of which take a long time to recover. The solution to repair it needs a special strategy. The aim is to get a high concentration of ATP (and the refilling of the creatine-phosphate tank), to undo the rigor bridges, which means forcing the metabolism producing more ATP than is spent.

This is not easy getting it resting (the reason why it takes several days) because muscular contraction is a main signal that stimulates ATP synthesis. However, this can be achieved by alternating resting with low gentle exercises (walking, push-ups, etc.) without forcing the machine, while at the same time enhancing the anaplerosis of the Krebs cycle, as we say in the combinations of nutrients of this invention.

**Physical aging**

Physical aging causes a general decrease in the ability to move, leading to a greater or lesser degree of disability. Although this decrease may be moderate in non-elderly people, it is significant in some jobs, such as police officers, firefighters, etc. It is especially serious in professional athletes, which forces them to abandon their career when they have the maximum technical preparation; many of them do not stop practicing their sport, but they do it in veteran competitions when they would like to continue as professional.

The main cause of the decrease in functional capacity has been traditionally considered a loss of muscle mass with age (sarcopenia). However, an equally important cause must be the deterioration of the passive mechanical system (particularly collagen and elastin), where the inherent lack of glycine, proline and lysine obviously worsens with age. This can be fought and avoided with the same nutritional treatments proposed in this invention, always taken on a regular basis, which should be done by all people from the stages of growth.

In addition, reinforcing the anaplerotic reactions of the Krebs cycle does improve the energy supply in all aspects mentioned above.

The best way to increase the Krebs cycle activity, as the main energy pathway, is to enhance its anaplerosis. As shown above (Fig. 8 and Table 2), the most effective feeders are *L*-aspartic acid (or aspartate, or their $K^{+}$-$Mg^{2+}$ salts) which could be taken as dietary supplements, as this invention proposes.

As to reduce the tropocollagen and tropoelastin cycles, in addition to glycine, proline and lysine should be added to the diet, as proposed here.

a-ketoglutarate (aKG), and vitamin C (VitC) are also required for collagen and elastin synthesis. All these products, or their precursors, are included in the combination of nutrients proposed in this invention.

In addition to its function in the synthesis of collagen and elastin, VitC plays a critical role in the respiratory chain, combating, as an antioxidant, oxygen toxicity; therefore.

For all its purposes, VitC should be taken daily, as a dietary supplement at enough amount.

As discussed above, the weakness of the mechanical system (collagen and elastin) is obviously a main cause of sports aging and injuries, and its reinforcement will help to solve or improve this issue. The solutions are therefore intakes of glycine, proline, lysine, and VitC added to the diet as nutritional supplements

The cause of discomfort during and after the play is an excess of rigor cross bridges by ATP spent higher than its production. This must not be considered normal and must be avoided as it can aggravate leading to more severe injuries or conditioning a large time of fatigue.

From the results showed in the previous section, we concluded that the minimum amounts of the mentioned products for daily intakes should be: glycine, 10 g; proline, 5 g; and lysine, 2.5 g.

The amount of VitC is estimated from the amount that the rat synthesizes. Making the relevant allometry calculations, it turns out to be, for a 70 kg man, between 1 and 2 g daily, (10-20 times higher than recommended). The intake of all these should be spaced two times to avoid their use for other general metabolic pathways.

As for aspartic acid (or aspartate), or citric acid (or citrate), based on calculations about plasm concentration and published data it is empirically stated that 3 g enhances energy by a moderately short time (about 20 minutes). This should be taking in this dose during the match, at these time intervals, or when possible.

**BRIEF DESCRIPTION OF THE DRAWINGS.**

**Fig. 1.** The Krebs cycle indicating points of anaplerosis (solid input arrows) and cataplerosis (dashed output arrows).

**Fig. 2.** Collagen synthesis, and the tropocollagen cycle.

**Fig. 3.** Structure of the sarcomere.

**Fig. 4.** Current model of sarcomere contraction.

**Fig. 5.** Relationship of the Krebs cycle with collagen synthesis.

**Fig. 6.** Porphyrin biosynthesis pathway.

**Fig. 7.** Design of the in vitro experiments to determine the main anaplerotic precursors of the Krebs cycle.

**Fig. 8.** Effect of various Krebs cycle feeders (or precursors thereof) on ATP synthesis.

**Fig. 9.** Model of sarcomere contraction, which answers the questions made in Fig. 4.

**Fig. 10.** Effect of the nutritional treatments according to programs 1, 2 and 3, on physical performance in professional and amateur athletes

**Fig. 11.** Increase of muscular mass in professional and amateur athletes with the invention's nutritional treatments.

**Fig. 12.** Effect of the nutritional treatments according to programs 1, 2 and 3, on the time required to recover from fatigue in professional and amateur athletes under (left) and over (right) 35 years of age.

**Fig. 13.** Effect of the nutritional treatments according to programs 1, 2 and 3, compared with the estimated recovery time with conventional treatments on the repair of recent physical injuries.

**Fig. 14.** Effect of the nutritional treatments according to programs 1, 2 and 3, on the repair of old physical injuries (between 2 and 24 months), which had not been fully resolved with conventional treatments.

**Fig. 15.** Decreased injury frequency in professional and amateur athletes after treatment.

Frequencies in the two to three years before the treatment, and two years after the treatment, according to the three nutritional programs.

**Fig. 16.** Decreased frequency of injuries in sedentary individuals after the nutritional treatment. Frequencies in the two to three years before treatment, and two years after treatment, according to the three nutritional programs.

## EXAMPLES

### Fitness - Aging

The study included three general objectives:

(1) To improve the physical fitness of professional and amateur athletes, which meant increasing their performance, reducing tiredness during the match, and subsequent fatigue time, in order to recover good fitness as soon as possible.

(2) To extent their activity time by fighting sports aging; and

(3) To repair physical injuries in both athletes and normal people. This group included two types: (a) recent injuries, (in the week or two weeks before consultation), and (b) old injuries (between six months and two years), which had not been fully resolved with conventional treatments because they continued to feel discomfort that made them move with poor performance due to physical inability or fear to have them again.

### Information to patients about nutrition and consent

The patients were asked to participate in this study, telling them that it was a nutritional treatment, all of whose products were accepted as such in the European Union, the United States, Canada, and many other countries, and that thus, athletes were authorized to take them regularly, because they were admitted by the anti-doping control of national and international competitions, including the Olympic Games. In fact, several professional athletes who took the formula of the invention with the indicated regularity, were subjected to this test in national and international competitions, passing them with sufficiency, showing us the official pass at their next appointment.

All of them were told that this treatment should be accompanied by a diet rich in protein (between 1.0 and 1.5 g of protein per kg of body mass each day) because, if they did not do so, metabolism would use the amino acids to meet other demands, and they would not have the desired effect. To this end, they were given a table of protein composition in common foods, so that they could choose according to their taste, and they were told the corresponding quantities according to their weight, making sure to take the indicated quantities.

They were told that they should complete it with one gram of vitamin C, in any galenic presentation, and the brand of their choice, daily, with breakfast.

**Fitness**

Athletes who wished to improve their fitness were told to continue their usual sporting activity in the same way, and their results were monitored every week on five variables: strength, velocity, achievements/effectiveness as improvement in performance (marks, aim, etc.), endurance without fatigue, and joint and muscle discomfort during and after exercise.

On the other hand, the recovery time from fatigue after a match to be ready for the next in optimal conditions. The meaning of these variables was explained to them, and they all agreed to check their variation with the treatment and showing the results.

Each of these variables was evaluated on a scale of 1 to 10, giving a value of 5 (50%) as starting point, and the average of the variables was calculated for each patient at each consultation. This group included 1,049 athletes (540 men and 509 women). Details of their specialty are given in Tables
5 (a and b).

**Table 5 (a).** Athletes under 35 years of age, according to their specialty

| Specialty | Men Professionals | | Amateurs | | Women Professionals | | Amateurs | |
|---|---|---|---|---|---|---|---|---|
| | Nm * | Age† | Nm * | Age† | Nm * | Age† | Nm * | Age† |
| Basket | **5** | **25.00**; 16-34; 6.11 | **17** | **23.77**; 14-34; 7.28 | - | - | **12** | **22.00**; 14-34; 6.35 |
| Canarian wrestling | **9** | **24.00**; 18-34; 5.90 | **8** | **22.43**; 16-34; 5.78 | - | - | - | - |
| Cycling | **18** | **24.08**; 18-34; 5.22 | **14** | **24.00**; 18-34; 5.48 | **6** | **23.00**; 18-34; 5.38 | **39** | **23.80**; 16-34; 6.46 |
| Gym | - | - | **25** | **24.33**; 18-34; 5.91 | - | - | **33** | **29.50**; 27-34; 5.92 |
| Karate | 6 | **23.83**; 17-34; 6.35 | **12** | **21.71**; 16-34; 6.16 | **4** | **21.80**; 16-29; 4.71 | **12** | **22.50**; 15-31; 6.69 |
| Hiking | - | - | **27** | **25.31**; 14-34; 6.84 | - | - | **37** | **24.67**; 18-34; 5.48 |
| Paddle tennis | - | - | **19** | **24.83**; 18-34; 5.30 | - | - | **12** | **27.33**; 21-34; 4.64 |
| Running | **12** | **21.62**; 14-34; 6.36 | **25** | **25.69**; 18-34, 5.84 | **8** | **24.14**; 18-34 6.01 | **27** | **25.17**; 18-34; 5.98 |
| Ski | - | - | **8** | **18.34**; 24.57; 5.82 | - | - | **6** | **24.60**; 19-33; 4.96 |
| Soccer | **9** | **23.00**; 18-31; **4**.69 | **23** | **22.60**; 16-34; 6.53 | **4** | **22.40**; 18-29; 4.08 | **7** | **21.75**; 14-32; 7.22 |
| Swimming | - | - | **27** | **25.90**; 18-34; 5.32 | - | - | **25** | **24.14**; 18-34; 5.38 |
| Tennis | - | - | **9** | **22.40**; 17-34; 6.50 | - | - | **8** | **24.50**; 18-33; 6.24 |
| Volleyball | - | - | **10** | **22.71**; 17-34; 6.18 | **28** | **23.30**; 18-29; 3.85 | **11** | **21.37**; 15-31; 5.50 |

(continued)

| Specialty | Men Professionals | | Amateurs | | Women Professionals | | Amateurs | |
|---|---|---|---|---|---|---|---|---|
| Weightlifting | - | - | - | - | **1** | **21.00**; 21; 0.00 | - | - |
| Total | **59** | **23.58;** 14-34; 1.05 | **22 4** | **23.38;** 14-34; 1.96 | **51** | **22.61;** 16-34; 1.02 | **22 9** | **24.27;** 14-34; 2.26 |

**Table 5 (b).** Athletes over 35 years old, according to their specialty

| Specialty | Men Professionals | | Amateurs | | Women Professionals | | Amateurs | |
|---|---|---|---|---|---|---|---|---|
| | Nm * | Age† | Nm * | Age† | Nm * | Age† | Nm * | Age† |
| Basket | **5** | **38.00**; 35-41; 2.28 | **24** | **41.33**; 35-48; 4.42 | - | - | **25** | **38.80;** 35-43; 3.19 |
| Cycling | **18** | **38.62**; 35-42; 2.68 | **22** | **46.67**; 35-56; 6.31 | | | **23** | **41.70**; 35-54; 6.43 |
| Gym | - | - | **27** | **50.33**; 37-65; 8.48 | - | - | **35** | **44.00**; 35-66; 9.49 |
| Karate | **8** | **43.00**; 35-53; 6.23 | **16** | **45.14**; 38-52; 5.28 | | | **9** | **39.60**; 37-42; 1.62 |
| Hiking | - | - | **23** | **45.45**; 39-67; 15.96 | - | - | **31** | **44.62**; 35-65; 9.60 |
| Paddle tennis | - | - | **25** | **39.17**; 35-52; 14.53 | - | - | **9** | **37.60**; 35-41; 2.15 |
| Running | **6** | **43.00**; 35-52; 6.32 | **11** | **38.67**; 35-45 3.50 | 12 | 43.12; 35-52; 5.67 | **18** | **39.00**; 35-43; 3.16 |
| Ski | - | - | **12** | **48.70**; 35-78; 12.72 | | | **16** | **44.80**; 38-52; 6.11 |
| Soccer | **3** | **38.33**; 36-41; 2.05 | **17** | **41.00**; 35-48; 4.60 | 2 | 38.50; 35-42; 3.50 | **5** | **38.50**; 35-41; 2.29 |
| Swimming | - | - | **19** | **49.50**; 35-69; 11.09 | - | - | **31** | **48.57**; 35-70; 11.77 |
| Tennis | - | - | **7** | **38.80**; 35-42; 2.56 | - | - | **4** | **43.00**; 35-52; 6.26 |
| Volleyball | - | - | **14** | **38.67**; 35-41; 2.05 | - | - | **9** | **38.50**; 35-41; 2.29 |
| Total | **40** | **40.19**; 35-34; 2.30 | **21 7** | 43.62; 35-78; 4.32 | 14 | 40.81; 16-34; 2.31 | **21 5** | **41.56**; 35-70; 3.28 |

* Nm: Number of participants
† Age: **mean** (in bold); range; SD

Muscle mass was estimated from body composition data, by anthropometric variables taken every week.

**Fatigue recovery**

This study involved all athletes, both those seeking to improve their physical condition and those with injuries that had healed, all of whom wished to reduce the time of fatigue after each long match. The study included 164 professional athletes (59 men, and 51 women under 35 years old, and 40 men and 14 women over 35 years old); and 885 amateur athletes (224 men, and 229 women under 35 years old), and 217 men and 215 women over 35 years old)

**Injury repair**

This group comprised athletes (professional and amateur), and normal people, all of whom had suffered injuries either in sports, or in their work requiring intense physical exercise, or from accidents in everyday life.
The group of recent injuries comprised a total of 229 patients: (108 men, 121 women). The group of old injuries comprised a total of 255 patients: (106 men, 149 women), Table 6(a, b).

**Table 6(a). Patients with recent injuries.** A total of 463 patients (195 men, and 268 women), according to the type of injury.

| Injury | Men | | | | Women | | | |
|---|---|---|---|---|---|---|---|---|
| | Number | Age (years) Mean | Range | SD | Number | Age (years) Mean | Range | SD |
| *Sprain* | 12 | 44.12 | 18-72 | 15,25 | 15 | 46.27 | 32-58 | 8.62 |
| *Muscle strain* | 9 | 47.86 | 22-70 | 15.46 | 12 | 40.44 | 14-68 | 16.79 |
| *Tendonitis* | 15 | 45.39 | 19-67 | 17.25 | 21 | 34.70 | 18-54 | 11.64 |
| *Muscle tear* | 25 | 39.27 | 20-65 | 14.64 | 17 | 45.70 | 20-65 | 14.34 |
| *Ligament tear* | 13 | 49.68 | 22-69 | 14.69 | 12 | 43.59 | 19-69 | 16.51 |
| *Tendon tear* | 9 | 45.00 | 20-65 | 14.36 | 5 | 28.80 | 22-38 | 6.49 |
| *Cartilage tear* | 14 | 33.70 | 20-45 | 8.87 | 14 | 40.74 | 18-63 | 15.68 |
| *Bone fracture* | 11 | 41.61 | 19-67 | 16.53 | 25 | 49.92 | 21-78 | 19.21 |
| **Total** | **108** | **43.33** | **18-72** | **15.75*** | **121** | **41.27** | **14-69** | **13.66*** |
| * Mean value | | | | | | | | |

**Table 6(b). Patients with old injuries** (between six months and two years), which had not been fully resolved with conventional treatments. A total of 311 patients (106 men and 205 women), according to the time of injury.

| Injury | Men | | | | Women | | | |
|---|---|---|---|---|---|---|---|---|
| | Number | Age (years) Mean | Range | SD | Number | Age (years) Mean | Range | SD |
| *Sprain* | 12 | 38.24 | 16-65 | 14.23 | 17 | 42.87 | 27-68 | 12.47 |
| *Muscle strain* | 8 | 41.18 | 19-72 | 16.72 | 19 | 35.23 | 18-55 | 14.53 |
| *Tendonitis* | 24 | 35.22 | 21-45 | 20.58 | 9 | 33.58 | 21-57 | 12.58 |
| *Muscle tear* | 15 | 41.35 | 18-63 | 12.75 | 27 | 37.15 | 22-69 | 10.83 |
| *Ligament tear* | 17 | 43.58 | 19-58 | 18.63 | 25 | 39.18 | 15-72 | 15.42 |
| *Tendon tear* | 5 | 41.88 | 22-63 | 23.54 | 12 | 31.75 | 20-41 | 9.58 |
| *Cartilage tear* | 8 | 29.54 | 21-58 | 18.75 | 17 | 38.64 | 17-65 | 14.38 |
| *Bone fracture* | 17 | 38.57 | 15-59 | 19.87 | 23 | 41.84 | 19-76 | 21.03 |
| **Total** | **106** | **38.69*** | **16-72** | **18.13*** | **149** | **37.53*** | **15-76** | **13.85*** |
| * Mean value | | | | | | | | |

Those with recent injuries were told that they should not dispense with conventional treatment, especially surgery, adding the nutritional supplement of the invention to achieve a better result in less time.
Patients who were taking drugs (analgesics/anti-inflammatories, or muscle relaxants) were told that they could

continue with those medications.

All were told that they should rest as much as possible, except for gentle physiotherapy, as long as it did not cause discomfort, and that they could do gentle exercises, as long as they did not have discomfort before, during and after. The usual training exercises should be reserved for when the pain or discomfort had completely ceased.

The repair of physical injuries was assessed by two procedures: (1) With the WOMAC questionnaire (pain, stiffness, and physical function), with the categorical version of five levels of response for each. The data were normalized in a range of values from 100 (the worst state of health) to 0 (the best). and (2) With the testimony of the affected people about their willingness to perform regular exercises, and the practice of sport -if applicable- without fear.

**Injury frequency**

The data in this section were taken from the data of all participants. All were asked about the injuries they had had in the last two years, their severity, and the time it took them to heal.

Those who wanted to improve their physical fitness and had no injuries when they started treatment were followed up on any injury they might have for two years after starting treatment. Those who came to heal injuries were followed up for two years after they were resolved. Considering that there were two risk groups of patients (higher in athletes, since they take more risks), they have been studied separately.

All patients volunteered to participate in this study with their informed consent, and agreed to strictly follow the given instructions, and to periodically report any changes they felt in the characteristics subject to this study, and any other symptoms they perceived during that time.

**Nutritional Programs - Formulas and Protocols**

Throughout this study three nutritional programs were designed, each one more complete than the previous in order, as the theoretical study progressed. When each program had a sufficiently large sample of patients, the next one was applied to new patients.

The composition of the products and their doses in each program were decided from the theoretical conclusions, and after checking them by doing several initial tests, arriving at the basic composition, with its doses for each program, which are shown in Table 7. The distribution of the patients to each nutritional program is shown in Tables 8 (physical fitness) and Table 9 (Injury repair).

**Table 7.** Composition of nutritional supplements to treat and prevent the health conditions related with physical exercise and physical injuries. Daily amounts and doses in the three designed programs. Daily amounts (grams) and doses*

| | Program 1 | | Program 2 | | Program 3 | |
|---|---|---|---|---|---|---|
| | Amount | Doses | Amount | Doses | Amount | Doses |
| Glycine | 10.0 g | 2 (5 g) | 10.0 g | 2 (5 g) | 10.0 g | 2 (5 g) |
| *L*-Proline | | | 5.0 g | 2 (2.5 g) | 5.0 g | 2 (2.5 g) |
| *L*-Lysine | | | 2.5 g | 2 (1.25 g) | 2.5 g | 2 (1.25 g) |
| *L*-aspartic/citric acid† | | | | | 12.0 g | 4 (3.0 g) |
| Vitamin C | 1.0 g | 1 (1.0 g) | 1.0 g | 1 (1.0 g) | 1.0 g | 1 (1.0 g) |

* All the products in powder form taken mixed, dissolved in yogurt, or thick juices (orange, tomato, etc.).
† *L*-Aspartic acid can be substituted by potassium-magnesium aspartate, or by citric acid, or potassium-magnesium citrate.

**Table 8. Distribution of athletes to increase physical fitness in the three nutritional programs.** Prog 1: glycine; Prog 2: glycine, *L*-proline, and *L*-lysine; Prog 3: glycine, *L*-proline, *L*-lysine and *L*-aspartic acid or citric acid, (or their potassium and magnesium salts).

| **Under 35 years old** | Men | | | | Women | | | |
|---|---|---|---|---|---|---|---|---|
| | Prog 1 | Prog 2 | Prog 3 | Total | Prog 1 | Prog 2 | Prog 3 | Total |
| Professional | 15 | 19 | 25 | 59 | 16 | 15 | 20 | 51 |
| Amateur | 50 | 72 | 102 | 224 | 73 | 75 | 81 | 229 |

(continued)

| **Under 35 years old** | Men | | | | Women | | | |
|---|---|---|---|---|---|---|---|---|
| | Prog 1 | Prog 2 | Prog 3 | Total | Prog 1 | Prog 2 | Prog 3 | Total |
| Total | 65 | 91 | 127 | 283 | 89 | 90 | 101 | 280 |

| **Over 35 years old** | Men | | | | Women | | | |
|---|---|---|---|---|---|---|---|---|
| | Prog 1 | Prog 2 | Prog 3 | Total | Prog 1 | Prog 2 | Prog 3 | Total |
| Professional | 10 | 13 | 17 | 40 | 5 | 3 | 6 | 14 |
| Amateur | 60 | 64 | 93 | 217 | 68 | 59 | 88 | 215 |
| Total | 70 | 77 | 110 | 257 | 73 | 62 | 94 | 229 |

**Table 9. Distribution of patients to repair physical injuries in the three nutritional programs:** Prog 1: glycine; Prog 2: glycine, proline and lysine; Prog 3: glycine, *L*-proline, *L*-lysine and *L*-aspartic acid or citric acid, (or the corresponding potassium and magnesium salts).

| **Recent injuries** | | | | |
|---|---|---|---|---|
| | Men | | | |
| | Prog 1 | Prog 2 | Prog 3 | Total |
| Men | 34 | 25 | 49 | 108 |
| Women | 41 | 36 | 44 | 121 |
| Total | 75 | 61 | 93 | 229 |
| **Old injuries** | | | | |
| | Men | | | |
| | Prog 1 | Prog 2 | Prog 3 | Total |
| Men | 27 | 38 | 41 | 106 |
| Women | 32 | 53 | 64 | 149 |
| Total | 59 | 91 | 105 | 255 |

The tests gave the following results:

(a) The daily reduction of each ingredient by half gave appreciable results, which became outstanding with the basic quantities.
(b) In some difficult to solve cases, especially injuries, the quantities of each component were doubled, or the same was maintained with double the number of doses, spaced out...
(c) The substitution of *L*-aspartic acid by potassium and magnesium *L*-aspartate gave similar results.
(d) For the alternative of replacing *L*-aspartic acid (or K-Mg *L*-aspartates) with citric acid (or K-Mg-citrate), 70% of the results showed the preference of *L*-aspartic acid or *L*-aspartates, and 25% of patients reported better results with citric acid or citrates, similar to those obtained with aspartic acid for the patients with aspartic acid in the same cases.

**Study duration and frequency of consultations**

The entire study lasted differently for each patient: the time needed to achieve the expected results, and a subsequent follow-up period.
During the treatment and follow-up period, all patients claimed to have met the protocol requirements, which was verified in periodic consultations.
The results were monitored with weekly consultations during the first month, twice a month during the following three months, and monthly during the rest of the treatment.
Once the objectives were achieved, the study was extended for two more years, with monthly consultations, to

monitor the maintenance of the results, and especially the frequency of injuries, during which the patients continued to follow the protocol.

In all cases, at each consultation, their weight, body fat and blood pressure were measured according to the general protocol. All these data had normal values, and there was no appreciable variation in body weight during the entire follow-up.

**Horses**

This group included six racehorses, all of which were fed program 3 (without vitamin C, as ascorbate is not a vitamin for horses). All were given the corresponding amount, according to their weight.

**RESULTS**

No side effects, e.g., gastric, or intestinal effects, or symptoms similar to seasickness that were observed with the treatments.

**Physical fitness**

Given the interest of these data for studying sports aging, those under and over 35 years of age were analyzed separately, the age considered the average limit for professional sport.

The analysis of the results showed that these variables improved in unison; some are explained because they depend on others. For example, although strength is specifically determinant of effectiveness in certain sports, such as weightlifting and all types of wrestling, it is also necessary for speed and effectiveness in other sports; endurance is inseparable from effectiveness in long-duration exercises, such as soccer, cycling, tennis, paddle tennis, baseball, American football, rugby, etc.). However, the relationship between some of them is not obvious; for example, endurance with effectiveness (although the first is useless without the second, but they could be considered independent). By presenting the results using the function of Eqn. (1), the effectiveness of the invention was demonstrated. The results are shown in figure 10. Increase of muscular mass is shown in Figure 11.

**Fatigue**

Fatigue time to full recovery after a long game lasting several hours, as soccer, tennis, paddle tennis, rugby, football, etc., usually takes several days; a week would be ideal, but this is often not possible. The results of the treatments presented in Figure 12 show a very large shortening of this time, both in athletes under 35 years of age and over this age, and in both men and women.

**Repair of physical injuries**

Unlike the previous ones, this study was not limited to athletes, but also included sedentary people. Three types of results are presented: (a) the repair of recent physical injuries; (b) the definitive repair of old injuries, supposedly cured with conventional treatments, but which had not been completely resolved because the patients continued to feel discomfort that made them move with poor performance due to physical incapacity or fear; and (c) the decrease in their frequency. The normal time to repair physical injuries, whether from sports or accidents (for example, hip injuries from falls, which was the most common case in sedentary women), is usually between two and four months or up to a year or more for the most serious cases.

The results are shown in figures 13 and 14. In both cases (recent and old injuries, respectively) program 1 (glycine only) gave good results in a reasonable time, according to the nature and severity of the condition; these results improved with program 2 (Gly, Pro, Lys), and became very good with program 3 (Gly, Pro, Lys, Asp/Citr).

**Frequency - Decrease in injuries**

Figures 15 and 16 shows the results on the frequency of injuries. Program 1 (Gly) gave acceptable results (30% reduction in injuries); Program 2 (Gly, Pro, Lys) gave better results, with an overall reduction of 46%, and better results (70% reduction) were obtained with Program 3 (Gly, Pro, Lys, Asp, or Citr).

Note that the risk of athletes that follow the treatment of the invention increases, since being stronger they could risk more.

The results of sedentary people were similar to those of athletes, maintaining the same percentages. This makes sense because the different fitness and risk in athletes and sedentary people is the same for each person.

It is important to point out that the time required to achieve the final result in all the cases mentioned corresponds to people who started the treatment at time zero on the graphs.

Athletes should be under the treatment described here as soon as possible, several weeks before the season; not wait, for example, until they are 35 years old, or tired, or injured, and should continue the treatment for a long time, at least throughout the season, although it should be fixed, like a habit. The same should be for sedentary because everyone will be interested in all these issues. All the products in the combination of the invention are nutrients, and there should be no suspension periods because food is necessary every day. The most preferred embodiment of the invention for the full purposes related here is shown in Table 10.

**Physical aging**

The increase in fitness corresponds to an increase in muscle mass, which is shown in Figure 11. An increase in muscle mass was seen in all patients. This was higher in young men, under 35-year age (6.5% with Program 3), than in older; however, in these people, the increase was also very significant, reaching 3.77%.

Similar, although slower during the first weeks and with a lower peak than that of men were seen in women, with respective values of 5.2% and 3.2%, respectively, as expected, given the difference in muscle mass between genders.

These results clearly demonstrate physical rejuvenation, or a way to fight sports aging through the nutritional treatment.

The average muscle masses before the treatment were 58% and 52% in <35 and >35 years old, respectively, in men; and 47% and 45% in <35 and >35 years old, respectively, in women.

The increase in muscle mass with program 3 (Fig. 10) in these groups were 6.5% and 3.8% in <35 and >35 years old, respectively, in men; and 5.2% and 3.2% in <35 and >35 years old, respectively, in women.

Thus, these increases mean increases of muscle mass of 3.77 and 1.98 kg in young and older men, respectively, and 2.5% and 1.44% in young and older women, respectively.

**Horses**

The six treated horses (all racers, all treated with program 3) are not enough to make a statistical study, but they are equally significant, considering the results in humans. All of them improved their strength and their marks, and their injuries were solved in a very short time.

The clearest case was a horse, which after the repair of a moderately severe injury with program 3 in one week, recovered full strength in 5 days (instead of the supposed 4 weeks).

## Claims

1. Use of a combination of glycine, *L*-proline, *L*-lysine, an acid selected from *L*-aspartic acid and citric acid or a combination thereof, and vitamin C, or a pharmaceutically acceptable salt thereof, in the prevention of health and performance conditions in physical exercises in a subject.

2. The use according to claim 1, wherein said health condition is selected from the group consisting of physical fitness, physical fatigue, and physical aging.

3. The use according to claims 1 or 2, wherein said salt of *L*-aspartic acid or citric acid is a potassium salt, or a magnesium salt.

4. The use according to any one of claims 1 to 3, where said combination comprises a dosage of 5.0-20.0 g/day of glycine, 2.5-10 g/day of L-proline, 1.25-5.0 g/day of *L*-lysine, 6.0-24.0 g/day of *L*-aspartic acid or 6.0-24.0 g/day of citric acid, and 1.0-2.0 g/day vitamin C, or of a pharmaceutically acceptable salt thereof.

5. The use according to any one of claims 1 to 4, wherein said subject is a mammal.

6. The use according to claim 5, wherein said mammal is human.

7. A combination of glycine, *L*-proline, *L*-lysine, an acid selected from *L*-aspartic acid and citric acid or a combination thereof, and vitamin C, or a pharmaceutically acceptable salt thereof, for use in the prevention or treatment of physical injuries in a subject.

**8.** The combination for use according to claim 7, wherein said subject is a mammal.

**9.** The combination for use according to claim 8, wherein said mammal is human.

**10.** A nutraceutical composition comprising the combination as defined in claim 4.

**11.** A pharmaceutical composition comprising the combination as defined in claim 4, and a pharmaceutically acceptable excipient.

**12.** A pharmaceutical composition according to claim 11, wherein said composition is a topical composition in form of cream, a gel, or a solution.

**13.** The pharmaceutical or nutraceutical composition according to claims 10 or 11, wherein said compositions are in the form of a crystalline powder.

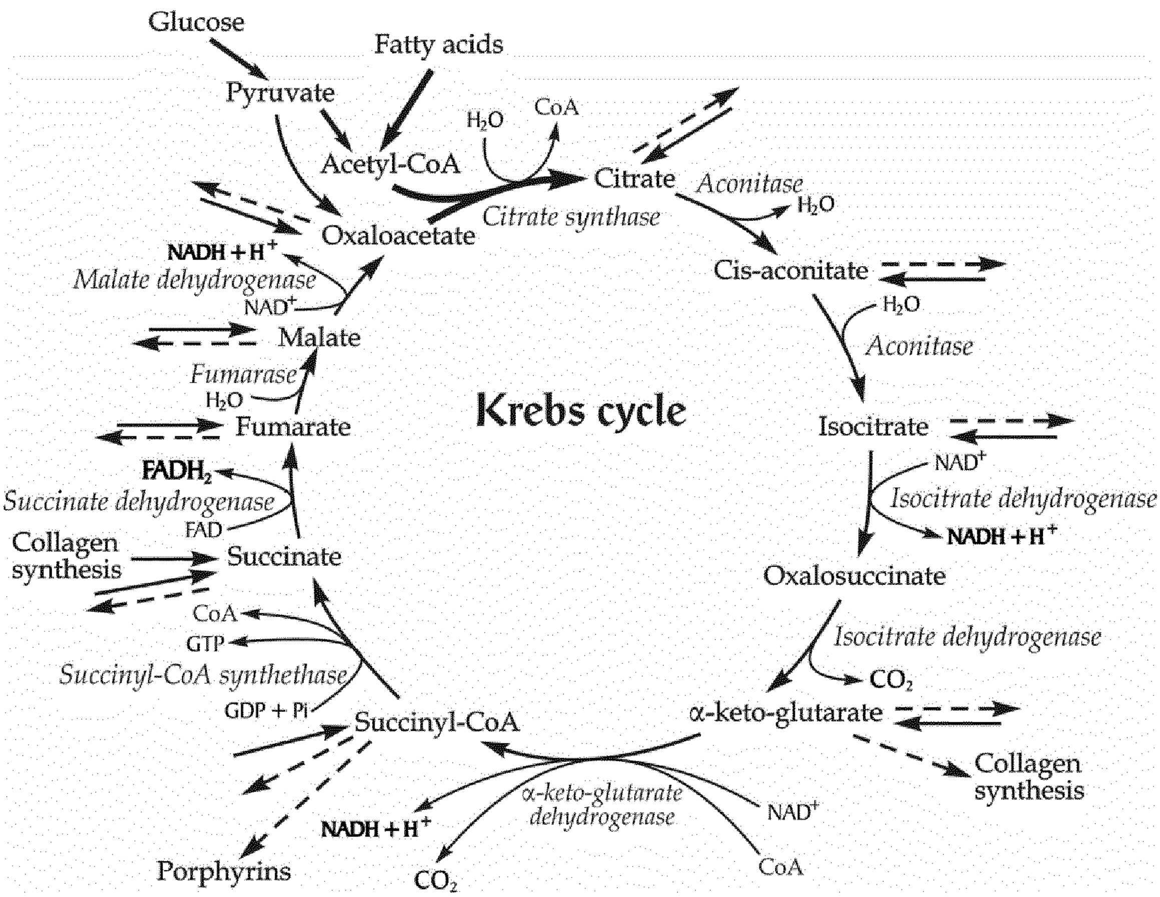
Glucose
Fatty acids
Pyruvate
Acetyl-CoA
$H_2O$
CoA
Citrate
Citrate synthase
Aconitase
$H_2O$
Oxaloacetate
NADH + $H^+$
Malate dehydrogenase
$NAD^+$
Cis-aconitate
$H_2O$
Aconitase
Malate
Fumarase
$H_2O$
Krebs cycle
Fumarate
Isocitrate
$NAD^+$
Isocitrate dehydrogenase
NADH + $H^+$
$FADH_2$
Succinate dehydrogenase
FAD
Collagen synthesis
Succinate
Oxalosuccinate
CoA
GTP
Succinyl-CoA synthethase
GDP + Pi
Isocitrate dehydrogenase
$CO_2$
α-keto-glutarate
Succinyl-CoA
Collagen synthesis
α-keto-glutarate dehydrogenase
NADH + $H^+$
$NAD^+$
Porphyrins
$CO_2$
CoA

Figure 1

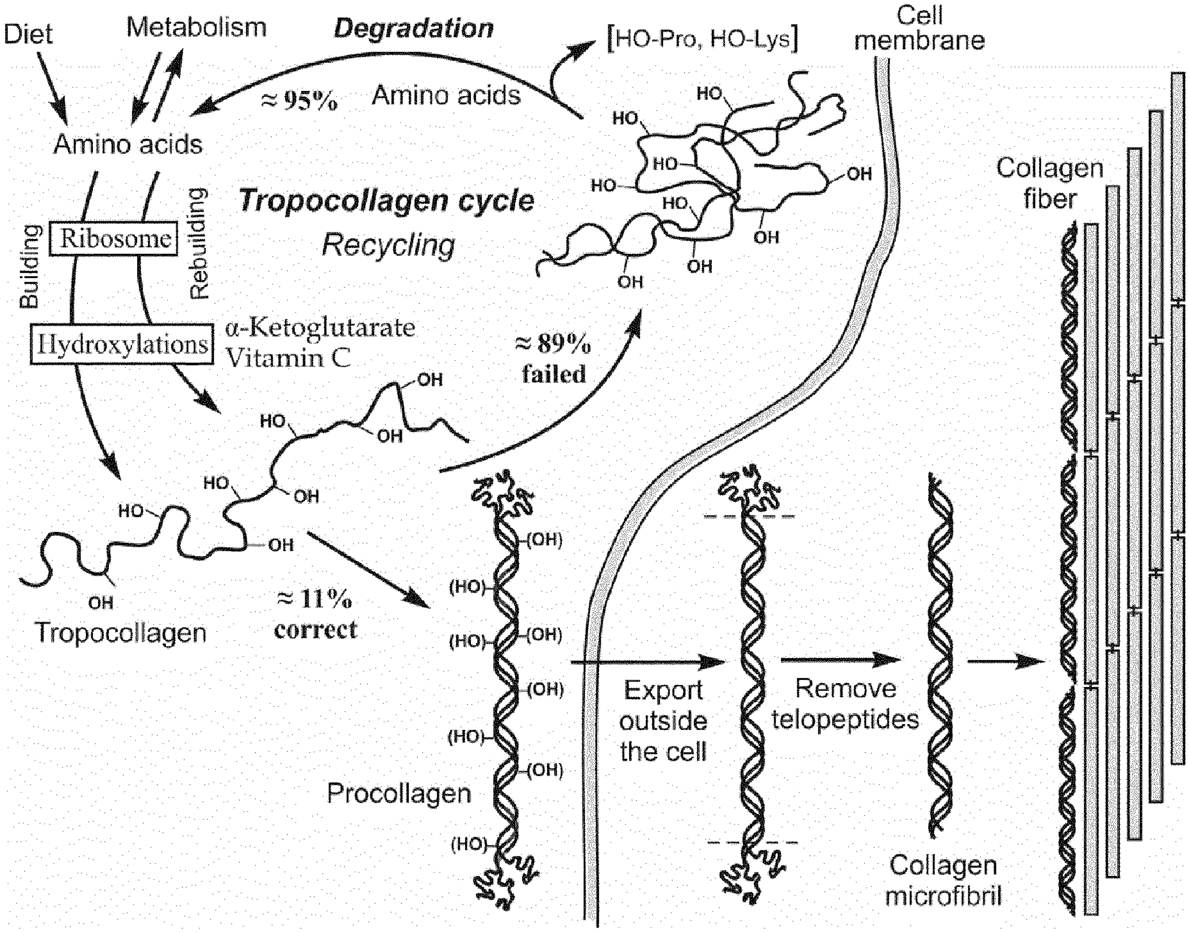


Figure 2

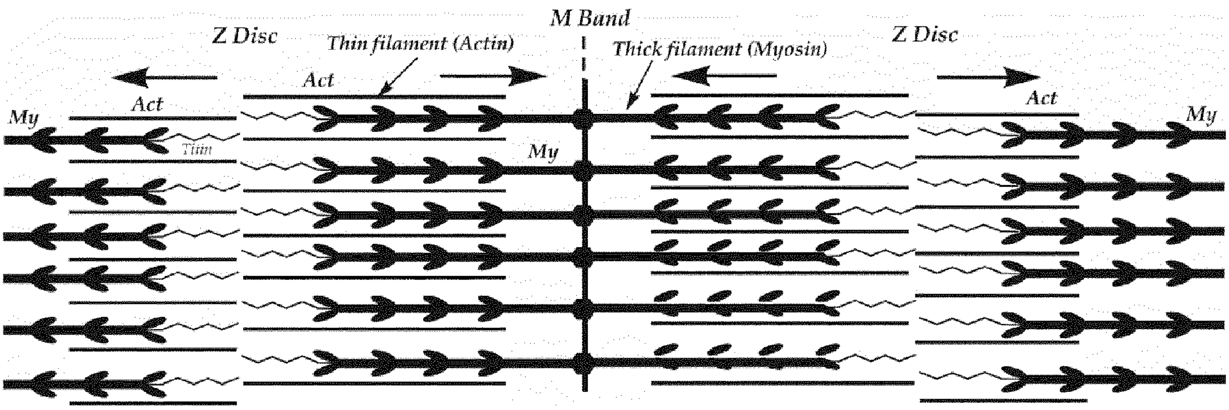


Figure 3

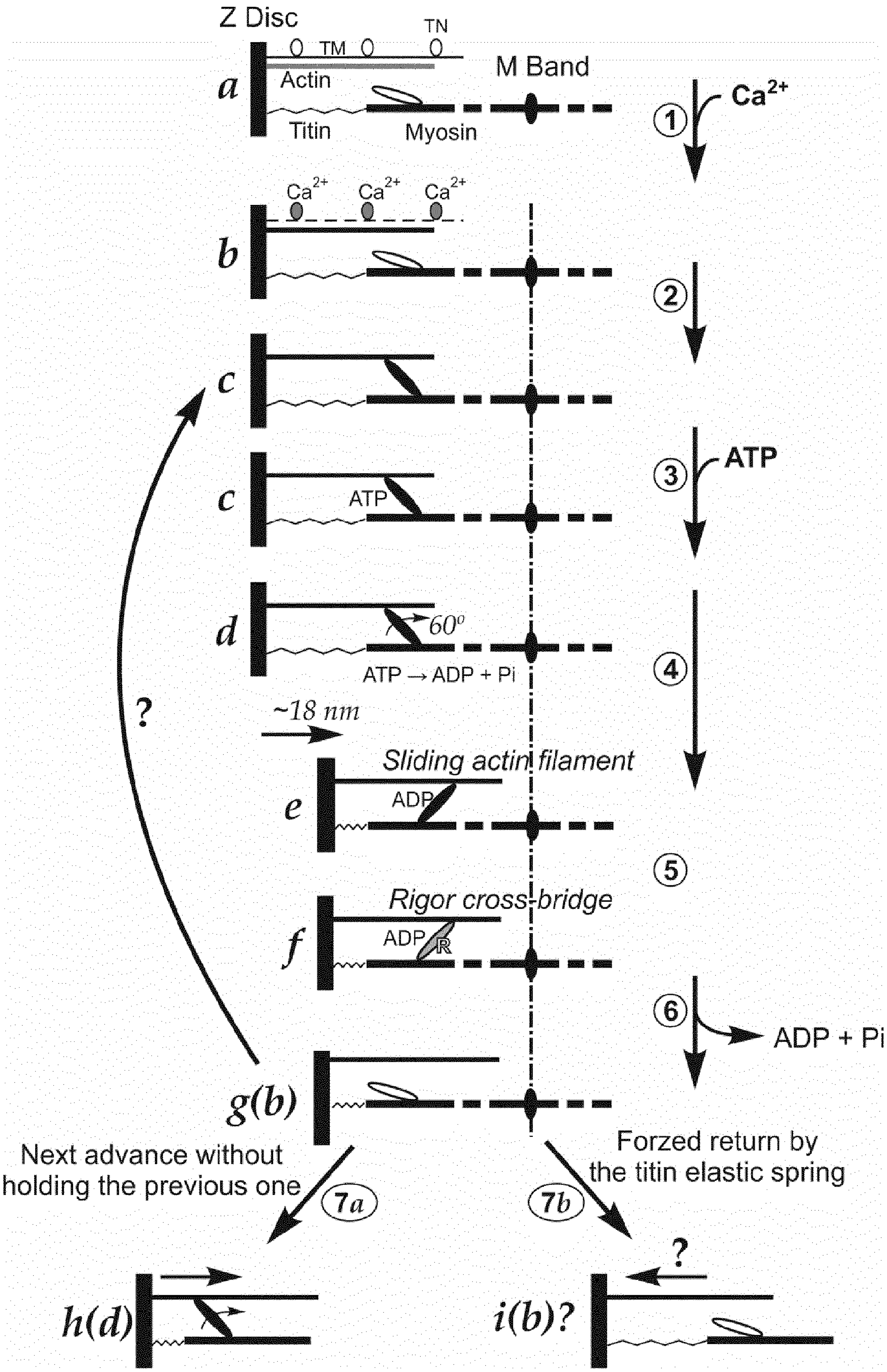
Z Disc
TN
TM
Actin
M Band
a
Titin
Myosin
① Ca2+
Ca2+ Ca2+ Ca2+
b
②
c
③ ATP
c
ATP
d
60°
ATP → ADP + Pi
④
?
~18 nm
Sliding actin filament
e
ADP
⑤
Rigor cross-bridge
f
ADP
⑥ ADP + Pi
g(b)
Next advance without
holding the previous one
7a
7b
Forzed return by
the titin elastic spring
?
h(d)
i(b)?

Figure 4

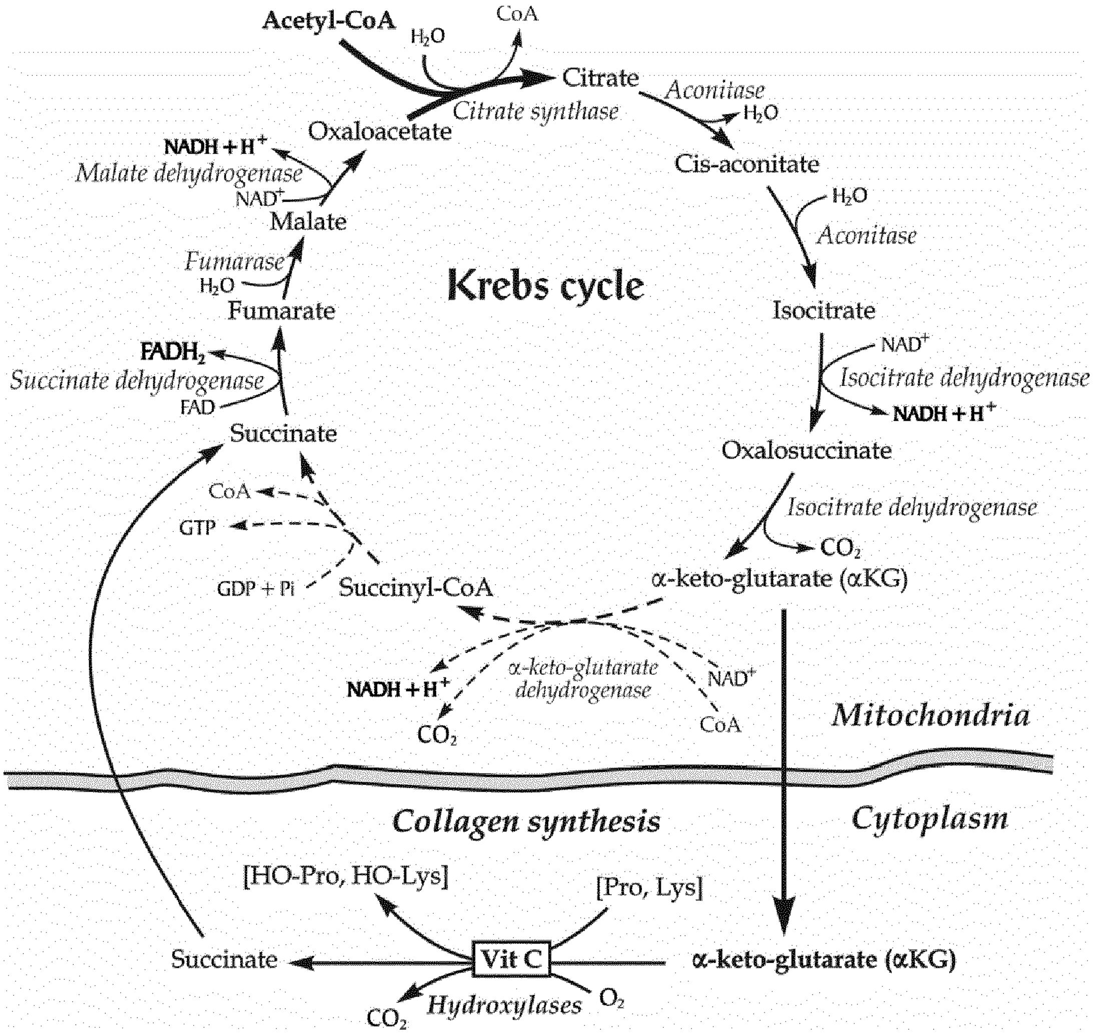
Acetyl-CoA
$H_2O$
CoA
Citrate
Citrate synthase
Aconitase
$H_2O$
Oxaloacetate
NADH + $H^+$
Malate dehydrogenase
$NAD^+$
Cis-aconitate
$H_2O$
Aconitase
Malate
Fumarase
$H_2O$
Krebs cycle
Fumarate
Isocitrate
$NAD^+$
Isocitrate dehydrogenase
NADH + $H^+$
$FADH_2$
Succinate dehydrogenase
FAD
Succinate
Oxalosuccinate
Isocitrate dehydrogenase
$CO_2$
CoA
GTP
GDP + Pi
Succinyl-CoA
α-keto-glutarate (αKG)
α-keto-glutarate dehydrogenase
NADH + $H^+$
$NAD^+$
$CO_2$
CoA
Mitochondria
Collagen synthesis
Cytoplasm
[HO-Pro, HO-Lys]
[Pro, Lys]
Succinate
Vit C
α-keto-glutarate (αKG)
$CO_2$
Hydroxylases
$O_2$

Figure 5

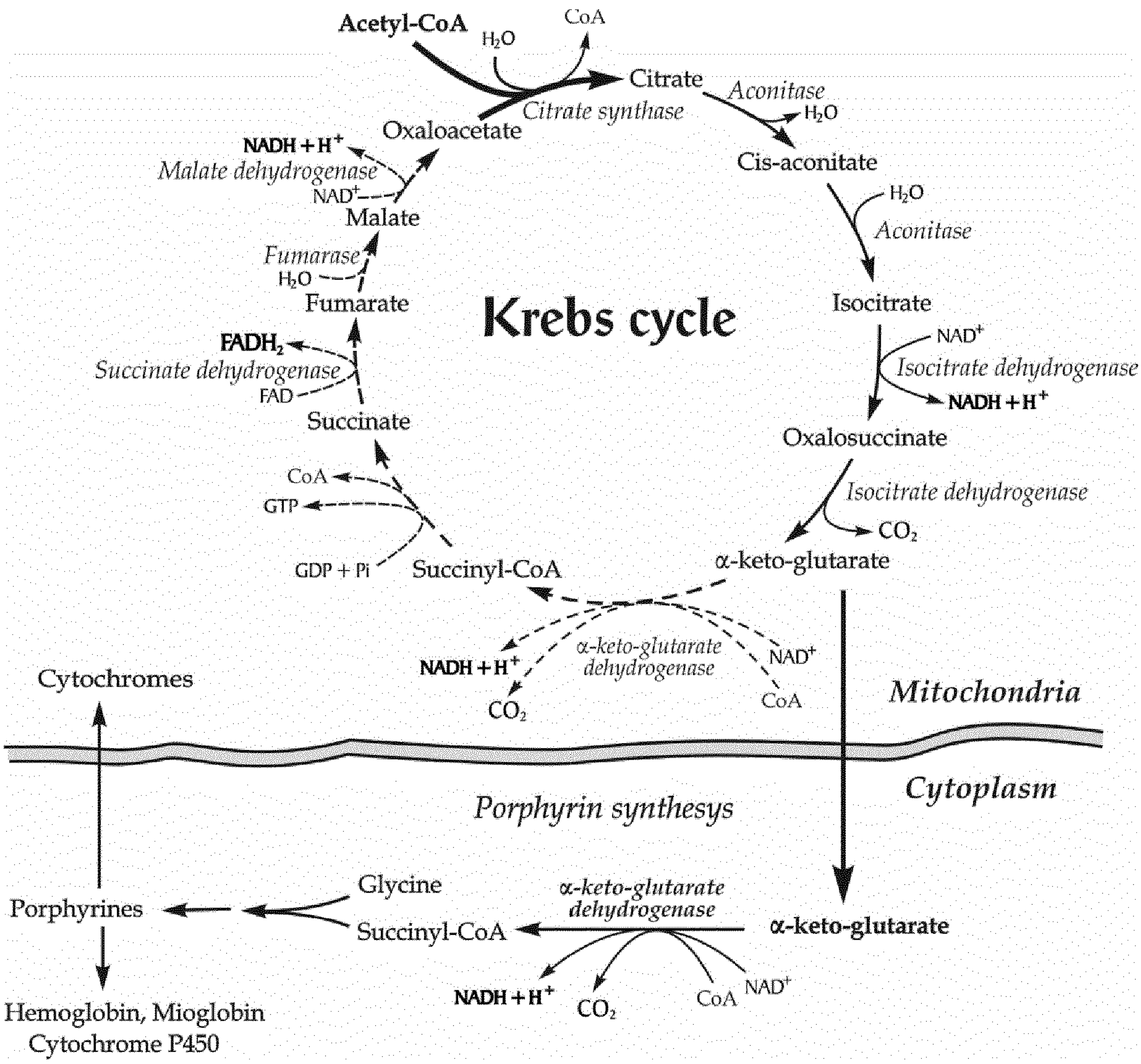
Acetyl-CoA
$H_2O$
CoA
Citrate
Citrate synthase
Aconitase
$H_2O$
Oxaloacetate
$NADH + H^+$
Malate dehydrogenase
$NAD^+$
Malate
Cis-aconitate
$H_2O$
Aconitase
Fumarase
$H_2O$
Fumarate
Krebs cycle
Isocitrate
$FADH_2$
Succinate dehydrogenase
FAD
$NAD^+$
Isocitrate dehydrogenase
$NADH + H^+$
Succinate
Oxalosuccinate
CoA
GTP
Isocitrate dehydrogenase
$CO_2$
GDP + Pi
Succinyl-CoA
α-keto-glutarate
α-keto-glutarate dehydrogenase
$NADH + H^+$
$NAD^+$
Cytochromes
$CO_2$
CoA
Mitochondria
Cytoplasm
Porphyrin synthesys
Glycine
α-keto-glutarate dehydrogenase
Porphyrines
Succinyl-CoA
α-keto-glutarate
$NADH + H^+$
$CO_2$
CoA
$NAD^+$
Hemoglobin, Mioglobin
Cytochrome P450

Figure 6

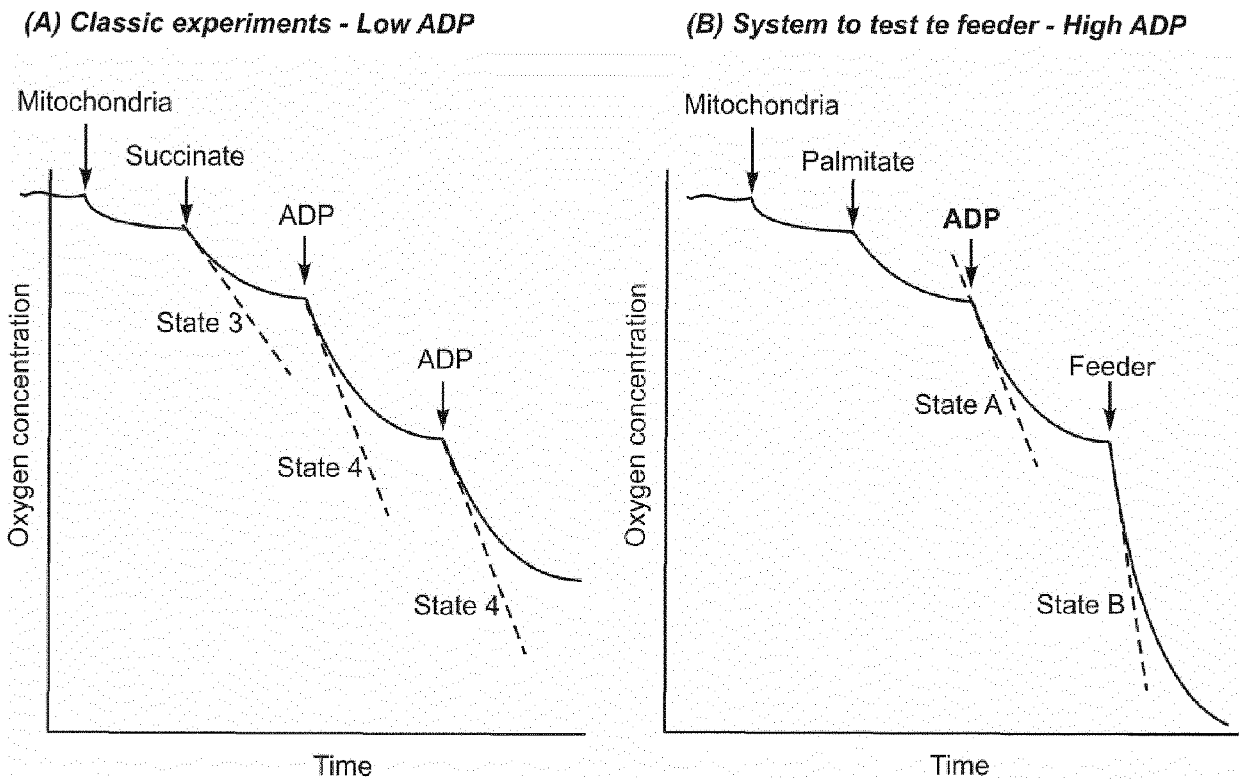


Figure 7

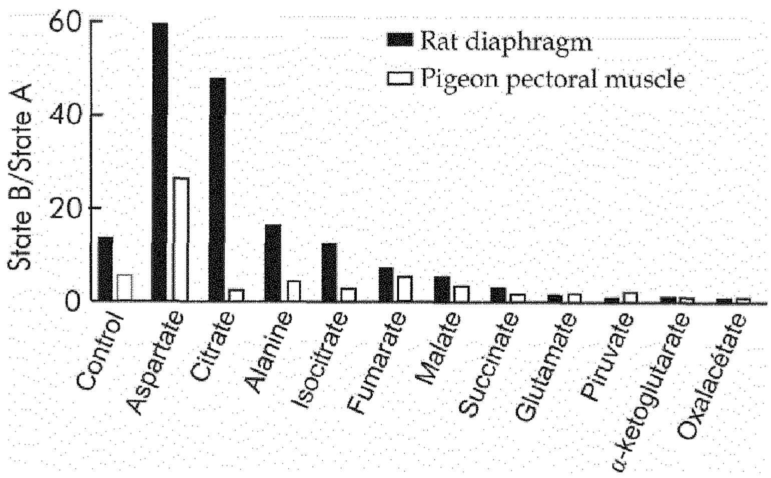


Figure 8

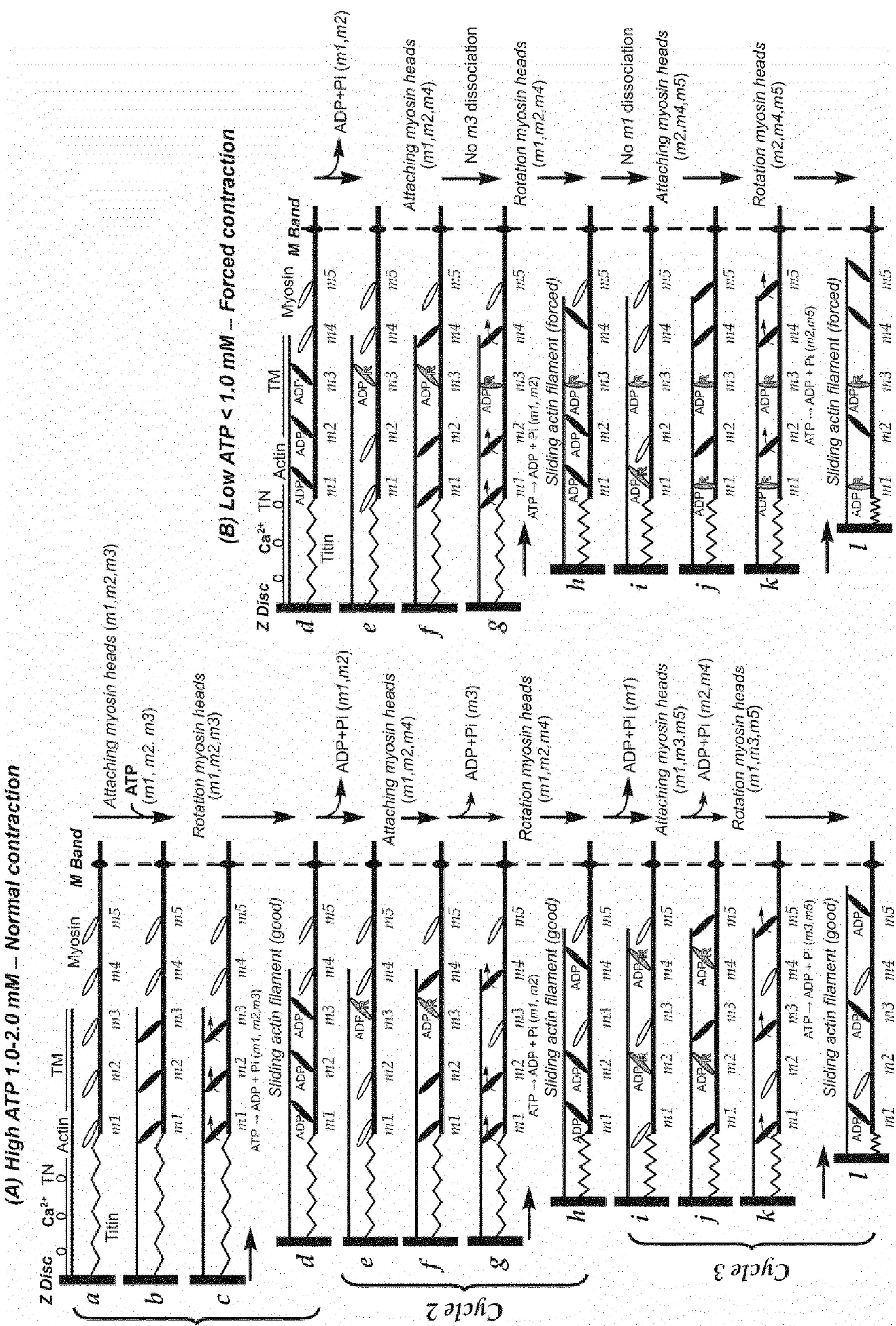
(A) High ATP 1.0-2.0 mM – Normal contraction
Z Disc
M Band
Ca²⁺
TN
Actin
TM
Myosin
Titin
Attaching myosin heads (m1,m2,m3)
ATP (m1, m2, m3)
Rotation myosin heads (m1,m2,m3)
ADP+Pi (m1,m2)
Attaching myosin heads (m1,m2,m4)
ADP+Pi (m3)
Rotation myosin heads (m1,m2,m4)
ADP+Pi (m1)
Attaching myosin heads (m1,m3,m5)
ADP+Pi (m2,m4)
Rotation myosin heads (m1,m3,m5)
Sliding actin filament (good)
Cycle 1
Cycle 2
Cycle 3
(B) Low ATP < 1.0 mM – Forced contraction
ADP+Pi (m1,m2)
Attaching myosin heads (m1,m2,m4)
No m3 dissociation
Rotation myosin heads (m1,m2,m4)
No m1 dissociation
Attaching myosin heads (m2,m4,m5)
Rotation myosin heads (m2,m4,m5)
Sliding actin filament (forced)

Figure 9

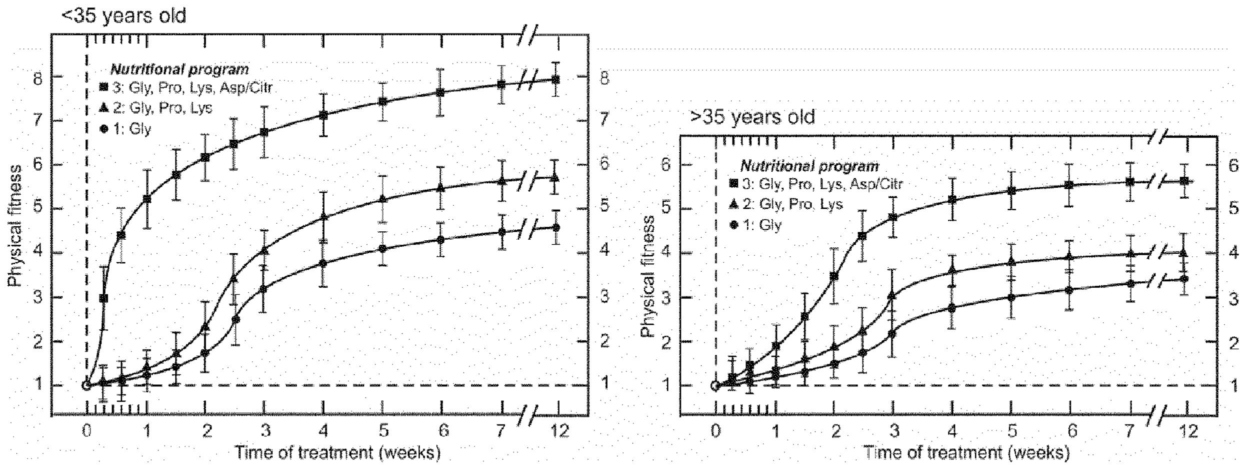


Figure 10

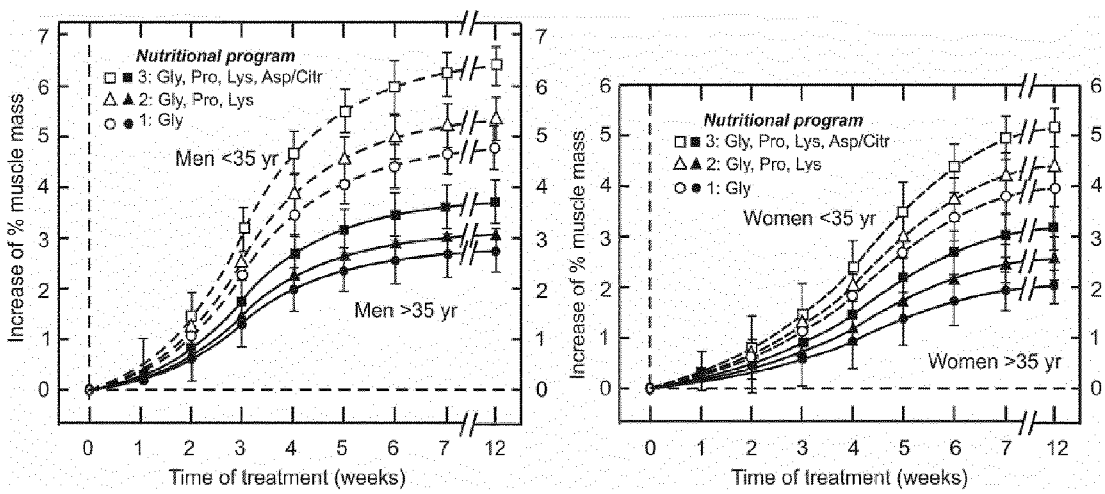


Figure 11

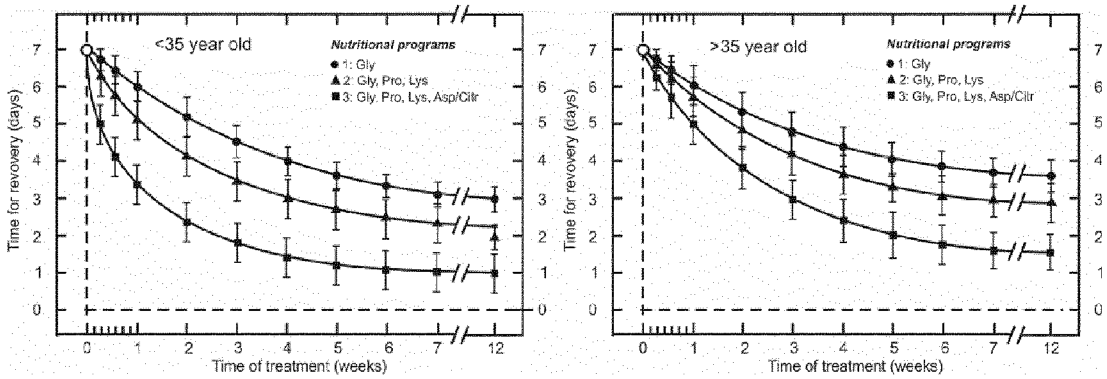


Figure 12

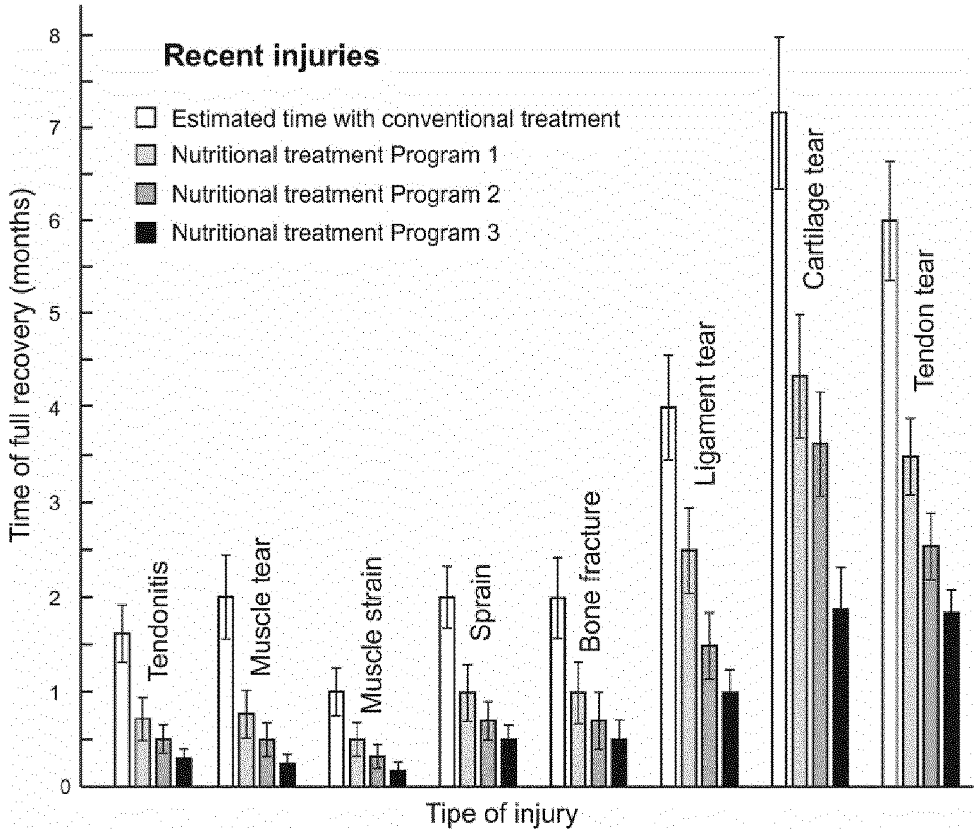


Figure 13

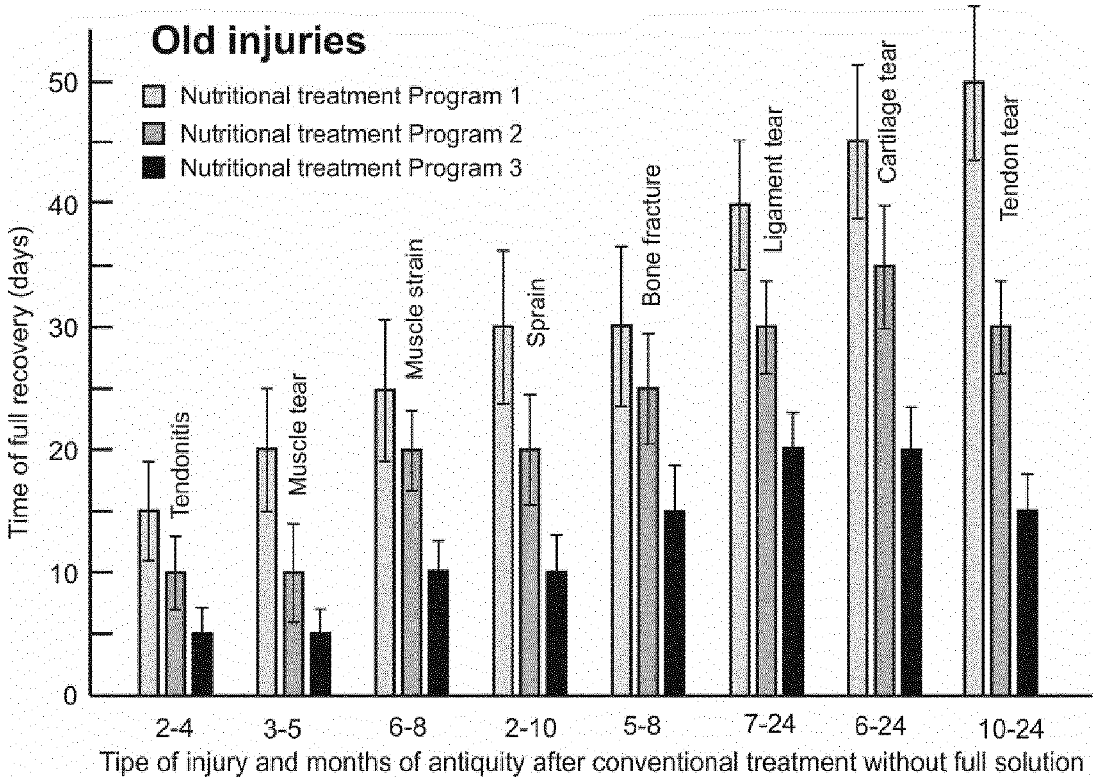


Figure 14

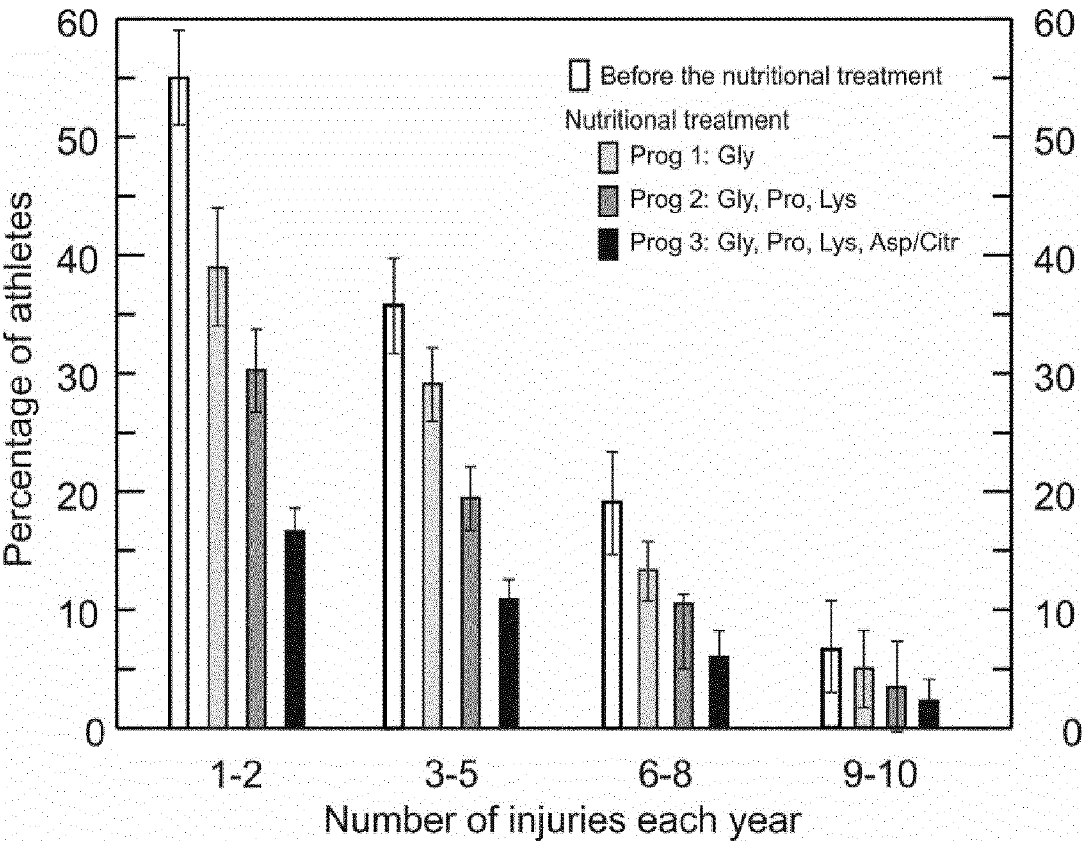


**Figure 15**

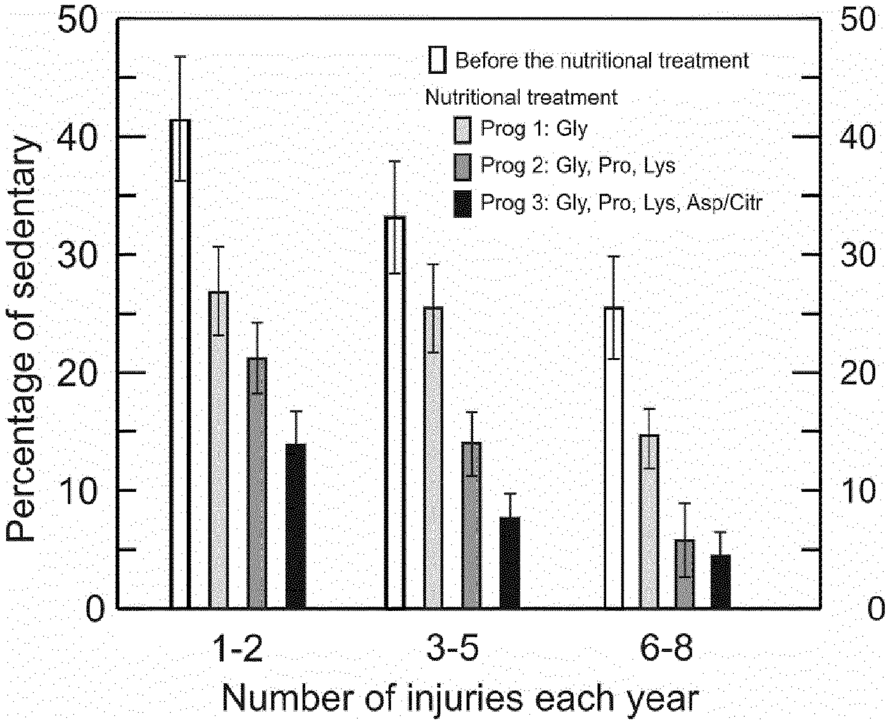


**Figure 16**

Europäisches Patentamt
European Patent Office
Office européen des brevets

# EUROPEAN SEARCH REPORT

Application Number
EP 24 38 3193

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WESSON MATTHEW ET AL: "Effects of Oral Administration of Aspartic Acid Salts on the Endurance Capacity of Trained Athletes", RESEARCH QUARTERLY FOR EXERCISE AND SPORT, vol. 59, no. 3, 1 September 1988 (1988-09-01), pages 234-239, XP093265461, US ISSN: 0270-1367, DOI: 10.1080/02701367.1988.10605509 Retrieved from the Internet: URL:https://dx.doi.org/10.1080/02701367.1988.10605509> | 1-6, 10-13 | INV. A61K31/194 A23L33/00 A61K9/00 A61K31/198 A61K31/375 A61K31/401 A61P3/02 A61P21/00 A61P43/00 |
| Y | * paragraph [0074]; claims; examples * ----- | 7-9 | |
| X | DATABASE GNPD [Online] MINTEL; 16 August 2006 (2006-08-16), anonymous: "Elixir", XP093265707, Database accession no. 572810 | 1-6, 10-13 | |
| Y | * the whole document * ----- | 7-9 | |
| X | DATABASE GNPD [Online] MINTEL; 28 August 2020 (2020-08-28), anonymous: "Daily Multivitamin", XP093265728, Database accession no. 8061965 | 1,3-6, 10-13 | |
| Y | * abstract * ----- | 7-9 | |
| | -/-- | | |

TECHNICAL FIELDS SEARCHED (IPC)

A61K
A23L
A61P

The present search report has been drawn up for all claims

1

| Place of search | Date of completion of the search | Examiner |
|---|---|---|
| Munich | 1 April 2025 | Moutafidou, Nikoleta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 24 38 3193

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE GNPD [Online] MINTEL; 7 February 2024 (2024-02-07), anonymous: "Vanilla Flavoured Vegan Protein Shake", XP093265758, Database accession no. 11465964 | 1,3-6, 10-13 | |
| Y | * abstract * | 7-9 | |
| Y | CN 116 391 870 A (TANG JIAQI) 7 July 2023 (2023-07-07) * claims; examples * | 7-9 | |
| E | WO 2024/223532 A1 (KASRAEE BEHROOZ [CH]) 31 October 2024 (2024-10-31) * claims * | 7-9 | |

TECHNICAL FIELDS SEARCHED (IPC)

The present search report has been drawn up for all claims

1

| Place of search | Date of completion of the search | Examiner |
|---|---|---|
| Munich | 1 April 2025 | Moutafidou, Nikoleta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

# ANNEX TO THE EUROPEAN SEARCH REPORT ON EUROPEAN PATENT APPLICATION NO.

EP 24 38 3193

This annex lists the patent family members relating to the patent documents cited in the above-mentioned European search report. The members are as contained in the European Patent Office EDP file on
The European Patent Office is in no way liable for these particulars which are merely given for the purpose of information.

01-04-2025

| Patent document cited in search report | Publication date | Patent family member(s) | Publication date |
| --- | --- | --- | --- |
| CN 116391870 A | 07-07-2023 | NONE | |
| WO 2024223532 A1 | 31-10-2024 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006056888 A2
- WO 2006087232 A1
- US 3009859 A, Laborit, H.


### Non-patent literature cited in the description


- **DVORAK J.** ; **JUNGE, A.** Football Injuries and Physical Symptoms. *American Journal of Sports Medicine*, 2000, vol. 28 (5), 3-9
- **ROBI, K.** ; **JAKOB, N.** ; **MATEVZ, K. et al.** The physiology of sports injuries and repair processes. *Current Issues in Sports and Exercise Medicine*, 2013
- **BAOGE, L.** ; **VAN DEN STEEN, E.** ; **RIMBAUT, S. et al.** Treatment of skeletal muscle injury: A review. *International Scholarly Research Network ISRN Orthopedics*, 2012
- **MELÉNDEZ-HEVIA, E.** ; **DE PAZ-LUGO, P.** ; **CORNISH-BOWDEN, A.** ; **CÁRDENAS, M. L.** A weak link in metabolism: the metabolic capacity for glycine biosynthesis does not satisfy the need for collagen synthesis. *Journal of Biosciences*, 2009, vol. 34, 853-872
- **DE PAZ-LUGO, P.** ; **LUPIÁÑEZ, J. A.** ; **SICILIA, J.** ; **MELÉNDEZ-HEVIA, E.** Control Analysis of collagen_synthesis by glycine, proline and lysine in bovine chondrocytes in vitro. - Its relevance for medicine and nutrition. *BioSystems*, 2023, vol. 232, 105004
- **COCHELLA, L.** ; **GREEN, R.** Fidelity in protein synthesis. *Curr. Biol.*, 2005, vol. 15, R536-R540
- **MELÉNDEZ-HEVIA, E.** ; **DE PAZ-LUGO, P.** Branch-point stoichiometry can generate weak links in metabolism: The case of glycine biosynthesis. *Journal of Biosciences*, 2008, vol. 33, 771-780
- **DE PAZ-LUGO, P.** ; **LUPIÁÑEZ, J. A.** ; **MELÉNDEZ-HEVIA, E.** High glycine concentration increases collagen synthesis by articular chondrocytes in vitro: acute glycine deficiency could be an important cause of osteoarthritis. *Amino Acids*, 2018, vol. 50, 1357-1365
- **WU, G.** ; **BAZER, F. W.** ; **BURGHARDT, R. C. et al.** Proline and hydroxyproline metabolism: implications for animal and human nutrition. *Amino Acids.*, 2011, vol. 40, 1053-1063
- **MILLER, B.F.** ; **OLESEN, J.L.** ; **HANSEN, L. et al.** Coordinated collagen and muscle protein synthesis in human patella tendon and quadriceps muscle after exercise. *J Physiol.*, 2005, vol. 567 (3), 1021-1033
- **MILLER, B. F.** ; **HANSEN, M.** ; **OLESEN, J. L. et al.** Tendon collagen synthesis at rest and after exercise in women. *J Appl Physiol*, 2007, vol. 102, 541-546
- **KJAER, M.** ; **JORGENSEN, N. R.** ; **HEINEMEIER, K.** ; **MAGNUSSON, S. P.** Exercise and regulation of bone and collagen tissue biology. *Progress in Molecular Biology and Translational Science*, 2015, vol. 135, 259-291
- **FATOUROS, I. G.** ; **JAMURTAS, A. Z.** Insights into the molecular etiology of exercise-induced inflammation: opportunities for optimizing performance. *Journal of Inflammation Research*, 2016, vol. 9, 175-186
- **OLSON K** ; **ZIMKA O** ; **PASIOROWSKI A et al.** Tiredness, fatigue, and exhaustion as perceived by recreational marathon runners. *Qualitative Health Research*, 2018, vol. 28, 1997-2010
- **BREMEL, R.** ; **WEBER, A.** Cooperation within Actin Filament in Vertebrate Skeletal Muscle. *Nature New Biology*, 1972, vol. 238, 97-101
- **MARUYAMA, K.** ; **WEBER, A.** Binding of adenosine triphosphate to myofibrils during contraction and relaxation. *Biochemistry*, 1972, vol. 11, 2990-2998
- **WEBER, A.** Actin binding proteins that change extent and rate of actin monomer-polymer distribution by different mechanisms. *Molecular and Cellular Biochemistry*, 1999, vol. 190, 67-74
- **PEMRICK, S.** ; **WEBER, A.** Mechanism of inhibition of relaxation by N-ethylmaleimide treatment of myosin. *Biochemistry*, 1976, vol. 15, 5193-5198
- **FRIDÉN, J.** Changes in human skeletal muscle induced by long-term eccentric exercise. *Cell Tissue Res*, 1984, vol. 236, 365-372

- **APPELL, HJ.** ; **SOARES, J.M.C.** ; **DUARTE, J.A.R.** Exercise, muscle damage and fatigue. *Sports Medicine*, 1992, vol. 13, 108-115
- **MCHUGH, M. P.** ; **CONNOLY, D. A. J.** ; **ESTON, R. G. et al.** The role of passive muscle stiffness in symptoms of exercise-induced muscle damage. *American Journal of Sports Medicine*, 1999, vol. 27, 594-599
- **FITTS, R. H.** The cross-bridge cycle and skeletal muscle fatigue. *J Appl Physiol*, 2008, vol. 104, 551-558
- **BROWNSTEIN, C. G.** ; **DENT, J. P.** ; **PARKER, P. et al.** Etiology and recovery of neuromuscular fatigue following competitive soccer match-play. *Frontiers in Physiology*, 2017, vol. 8, 1-14
- **CONSTANTIN-TEODOSIU, D.** ; **CONSTANTIN, D.** Molecular mechanisms of muscle fatigue. *Int. J. Mol. Sci.*, 2021, vol. 22, 11587
- **NAIR, K. S.** *Aging muscle Am. J. Clin. Nutr.*, 2005, vol. 81, 953-963
- **FIGUEIREDO, P. A.** ; **MOTA, M. P.** ; **APPELL, H. J.** ; **DUARTE, J. A.** The role of mitochondria in aging of skeletal muscle. *Biogerontology*, 2008, vol. 9, 67-84
- **KWAK, H.-B.** Aging, exercise, and extracellular matrix in the heart. *Journal of Exercise Rehabilitation*, 2013, vol. 9, 338-347
- **GUMPENBERGER, M.** ; **WESSNER, B.** ; **GRAF, A. et al.** Remodeling the skeletal muscle extracellular matrix in older age-effects of acute exercise stimuli on gene expression. *International Journal of Molecular Sciences*, 2020, vol. 21, 7089
- **MELÉNDEZ-HEVIA, E.** ; **DE PAZ-LUGO, P.** ; **SÁNCHEZ, G.** Glycine can prevent and fight virus invasiveness by reinforcing the extracellular matrix. *Journal of Functional Foods*, 2021, vol. 76, 104318
- **WESSON, M.** ; **MCNAUGHTON, L.** ; **DAVIES, P.** ; **DAVIES, P.** Effects of oral administration of aspartic acid salts on the endurance capacity of trained athletes. *Research Quarterly for Exercise and Sport*, 1988, vol. 59, 234-239
- **MAUGHAN, R. J.** ; **SADLER, D. J. M.** The effects of oral administration of salts of aspartic acid on the metabolic response to prolonged exhausting exercise in man. *Int J Sports Med*, 1983, vol. 4, 119-123
- **TRIPLETT, N. TRAVIS** ; **STONE, MICHAEL H.** ; **ADAMS, CHIP** ; **ALLRAN, KEVIN D.** ; **SMITH, TIM W.** Effects of aspartic acid salts on fatigue parameters during weight training exercise and recovery. *Journal of Applied Sport Science Research*, 1990, vol. 4, 141-147
- **AHLBORG, B.** ; **EKELUND, L.G.** ; **C.G. NILSSON**. Effect of potassium-magnesium-aspartate on the capacity for prolonged exercise in man. *Acta Physiol. Scand.*, 1968, vol. 74, 238-245
- **GUPTA, J. S.** ; **SRIVASTAVA, K. K.** Effect of potassium-magnesium aspartate on endurance work in man. *Indian J. Exp. Biol.*, 1973, vol. 11, 392-394
- **BARNES, R. H.** ; **ALCAZAR LABADAN, B.** ; **SIYAMOGLU, B.** ; **ROBERT B.** ; **BRADFIELD, R. B.** Effects of exercise and administration of aspartic acid on blood ammonia in the rat. *Am. J. Physiol.*, 1964, vol. 207, 1242-1246